# EUROPEAN PATENT APPLICATION

(11) **EP 1 918 255 A1**
(43) Date of publication of application: **07.05.2008**
(21) Application number: 06766786.5
(22) Date of filing: 15.06.2006
(51) Int. Cl.: C02F 3/34, C02F 3/00, C02F 3/10

(54) **METHOD OF FEEDING MICROBIAL ACTIVITY CONTROLLING SUBSTANCE, APPARATUS THEREFOR, AND MAKING USE OF THE SAME, METHOD OF ENVIRONMENTAL CLEANUP AND BIOREACTOR**

(30) Priority: 15.06.2005 JP 2005175809; 02.05.2006 JP 2006128641
(71) Applicant: Central Research Institute of Electric Power Industry, Tokyo 100-8126 (JP)
(72) Inventor: UEMOTO, Hiroaki c/o CENTRAL RESEARCH INSTITUTE, Res earch Laboratory; 1646, Abiko (JP); MORITA, Masahiko c/o CENTRAL RESEARCH INSTITUTE, Re search Laboratory; 1646, Abiko, (JP); WATANABE, Atsushi c/o CENTRAL RESEARCH INSTITUTE, R esearch Laboratory; 1646, Abiko, (JP)
(74) Representative: Neilson, Martin Mark
(86) International application number: PCT/JP2006/312073
(87) International publication number: WO 2006/135028

(57) **Abstract**

Without using any pumps and controlling devices, to control microorganism's activity by supplying an activity controlling substance which is necessitated for the microorganism activity is realized. A microbial activity controlling material 3 is filled into a vessel 4 having a sealed structure and at least a part of which is provided with a non-porous film 2, and then, the microbial activity controlling material 3 is supplied through the non-porous film 2 part of the vessel 4 to ambient places around the vessel 4 at the speed controlled by the molecular permeation performance of the non-porous film 2, and thereby the activities of microorganisms residing around the vessel is controlled. The microbial activity controlling material 3 is at least one of materials which function as electron donor being of an energy source of microorganism, acidic materials, basic materials, inorganic salts, oxygen releasing materials and oxygen absorbing materials, except combinations of acidic materials and basic materials and combinations of oxygen releasing materials and oxygen absorbing materials.

## Description

### TECHNICAL FIELD

This invention relates to a method for supplying a microbial activity controlling material, an apparatus therefor, as well as an environmental purification method and a bioreactor both using thereof. More particularly, this invention relates to a method for supplying a microbial activity controlling material on an effective removal of environmental pollutant existing in a region to be decontaminated by a biological treatment using a microorganism, an apparatus therefor, as well as an environmental purification method and an bioreactor both using thereof.

### BACKGROUND ARTS

On practicing a biological treatment using a microorganism, organic matters, such as alcohol, should be sometimes supplied to the system for the process as an electron donor being of energy source for the microorganism. For instance, with respect to a sewerage treatment process wherein nitrogen compounds such as ammonia existing in a liquid to be treated are removed by the biological treatment, methanol is supplied as the electron donor being of energy source for the microorganism at a volume as much as needed through a pump which is controlled by a controlling unit, in order to accelerate denitrification reaction after nitrification reaction (See, Patent Literature 1).

In addition, with respect to a bioreactor for removing nitrogen, which is the type where a sheet polymer gel on which a microorganism effective for removing nitrogen compounds such as ammonia existing in a liquid to be treated comes into contact with the liquid to be treated at one side thereof, and also comes into contact with an electron donor being of energy source for the microorganism at other side thereof (See, Patent literatures 2 and 3), alcohol is supplied as the electron donor being of energy source for the microorganism. The supplying of the alcohol is performed by using a circulating pathway which is formed by connecting an interior space of the polymer gel support formed in the bioreactor with an alcohol reservoir tank via piping, and by operating a circulation pump, various valves, instruments, etc., so as to control the timing for supplying the alcohol and the volume of the alcohol.

Here, when the alcohol is supplied in excess amounts, the alcohol can cause a degression in water quality, because its amount is too large for the microorganism to consume it completely, and which results in the residual alcohol in water or so. Conversely, when the alcohol is supplied too little, the nitrate concentration increases, because the energy source becomes insufficient and thus the denitrification reaction causes at an inadequate level. Therefore, with respect to the supplying of alcohol, the volume and the timing of supplying it are controlled by using pumps, etc., so that alcohol diluted to about 10 % is supplied from the alcohol reservoir tank.

Patent Literature 1 : Japanese Patent No. 3260554
Patent Literature 2 : Japanese Patent No. 3340356
Patent Literature 3 : Japanese Patent No. 2887737

### DISCLOSURE OF THE INVENTION

### PROBLEMS TO BE SOLVED BY THE INVENTION

Therefore, the methods for supplying electron donor to a microorganism in the prior arts' bioreactor require a series of equipments such as pumps and controlling devices for maintaining the supplying volume of alcohol solution, such as methanol or ethanol, which is an electron donor as an energy source for denitrifying bacteria, to a constant level. Thereby, system becomes large and the operation of the system becomes complicated, and which is also followed by a rise of running cost.

Further, because when the alcoholic concentration of the alcohol solution such as methanol or ethanol solution is high and this solution is contacted directly to microorganism, the microorganism may die, there is a troublesomeness that the solution must be diluted with water to adjust its concentration to a level at which the microorganism does not die. In addition, since the undiluted alcohol solution can not be used directly, it must be reserved in a diluted form, and thus reservoir tank to be used becomes larger for that. Still more, since in the methods for supplying electron donor to a microorganism in the prior arts' the diluted alcohol per se was supplied to the microorganism, there is a problem that a waste alcohol including a large volume of impurities or the like can not be applied to the method. For instance, when a waste alcohol produced from purification process of green tea is used as an energy source, because it contains catechin, a fear that the microorganism comes to die will arise, even if the waste alcohol is used in a diluted form. Therefore, to remove the impurities is necessitated, and thus, the reuse of the waste alcohol is substantially impossible.

Further, with respect to the nitrogen removing bioreactor which is shown as in Patent Literatures 2 and 3, and which is of the type in which electron donor as the necessitated energy source is contacted to a space surrounded by microorganism - immobilized sheet polymer gels, it is required that, at a volume that is not flow over from the reactor, the alcohol should be supplied through a supplying pipe to the reactor. The larger the reactor become, the more difficult to distribute alcohol uniformly throughout the reactor. Thus, it is difficult to scale up the reactor.

In addition to the point of view of such electron donor supplying, on a biological treatment using microorganism, it is important for proceeding the biological treatment efficiently to prepare an environment where the microorganism can be activated easily. Thus, it is desired to establish a brief procedure to supply to the microorganism various materials for preparing an environment where the microorganism can be activated easily

Therefore, the present invention aims to provide a brief method and apparatus for realizing the control of microorganism's activity by supplying an activity controlling substance which is necessitated for the microorganism activity, without using any pumps and controlling devices. The present invention also aims to provide a method for supplying activity controlling material to microorganism, and apparatus therefor, capable of scaling up the reactor. The present invention also aims to provide a method for supplying activity controlling material to microorganism, and apparatus therefor, capable of reuse of waste alcohol as energy source.

Further, the present invention aims to provide a bioreactor which is compact one and which is no need for controlling of supplying of activity controlling agent to the microorganism. In addition, the present invention aims to provide a bioreactor for removing efficiently environment polluting substances such as ammonia, nitrate ion, and nitrite ion, existing in a region to be treated and being of liquid phase, gas phase or solid phase.

Furthermore, the present invention aims to activate only a particular microorganism under a certain environment. The present invention aims to provide a method for environmental purification or soil improvement by controlling the activity and multiplication of microorganisms existing in a decontamination targeting area, and activating and colonizing selectively a certain microorganism desired to be functionalized.

### MEANS FOR SOLVING THE PROBLEMS

In order to attain to such subjects as mentioned above, we, the inventors, have paid attention to molecular permeability of a non-porous film, namely, a fact that when a polyethylene film which is a non-porous film is subjected to contact with an undiluted solution of methanol or ethanol on one side thereof, methanol molecules or ethanol molecules can be gently supplied from the opposite side of the film. Then, we have found that it is possible to supply a material to microorganism with a gentle sustained-release, wherein the material functions as an electron donor, and is of an energy source for microorganism, by utilizing such a molecular permeability of the non-porous film. Further, we have found that the activity of the microorganism can be controlled by supplying gently various materials necessitated for activity of the microorganism, to the microorganism, with utilizing such a molecular permeability of the non-porous film and subjecting the materials to permeate through the film. Finally, we have reached the present invention.

The method for supplying a microbial activity controlling material according to the present invention which is based on the above mentioned knowledge comprises filling a microbial activity controlling material into a vessel having a sealed structure and at least a part of which is provided with a non-porous film, supplying the microbial activity controlling material through the non-porous film part of the vessel to ambient places around the vessel at the speed controlled by the molecular permeation performance of the non-porous film, and thereby controlling the activities of microorganisms residing around the vessel. Further, the apparatus for supplying a microbial activity controlling material according to the present invention which is based on the above mentioned knowledge comprises a microbial activity controlling material and a vessel having a sealed structure and at least a part of which is provided with a non-porous film, wherein the microbial activity controlling material is filled into the vessel, and the material is supplied through the non-porous film part of the vessel to ambient places around the vessel at the speed controlled by the molecular permeation performance of the non-porous film, and thereby the activities of microorganisms residing around the vessel are controlled.

The microbial activity controlling material filled in the vessel is released gently at the speed controlled by the molecular permeation performance of the non-porous film. Thus, by adjusting the permeation rate of the microbial activity controlling material through the non-porous film with varying the material of film, the thickness of film, the density of film, or the like, which are elements for deciding the molecular permeation performance of the non-porous film, the microbial activity controlling material can be supplied at a gentle rate constantly, and thus the activity of microorganism can be controlled.

Therefore, there is no need of providing with a system for maintaining the supplying volume of the microbial activity controlling material constantly, and thus it is possible to repress greatly the cost for system and the running cost as compared with the case where the supplying volume is controlled by pumps and controlling devices as in the prior art's cases. In addition, when the whole apparatus consists of the non-porous film, the microbial activity controlling material can be released gently through the wide and whole face of the non-porous film, and thus it can be supplied throughout the bioreactor. Therefore, it is possible to scale up the bioreactor.

Further, even when a germicidal microbial activity controlling material which may bring extinction to the microorganisms on directly contacting with the material, such as an undiluted alcohol, is adapted, it is possible to supply it in a diluted form by repressing the permeating rate per unit area to an adequately low level, thus the extinction of microorganisms on using the undiluted alcohol can be prevented. In addition, it is possible to omit the process which is needed for the prior art's methods and where the alcohol is diluted with water to a concentration of not inducing the extinction of the microorganisms, so as to save the operator's labor, and also possible to supply alcohol for a prolonged time with a same volume of liquid.

Incidentally, as the microbial activity controlling material, electron donor materials, acidic materials, basic materials, inorganic salts, oxygen, oxygen releasing materials and oxygen absorbing materials are enumerated, and they are used singly or any combination of two or more kinds of them. However, any combination of a certain acidic material and a certain basic material should be excluded because if they are concurrently used, a neutralization reaction is caused within the vessel as a matter of course, and which results in the failure of pH control around the vessel. In addition any combination of certain oxygen releasing material and a certain oxygen absorbing material should be also excluded because if they are concurrently used, the supplying of oxygen to the region around the vessel and the absorption of oxygen from the region around the vessel can not be performed anymore. However, in the cases that they are installed separately to mutually independent vessels, such exclusion is out of the range.

When a single kind of material is used as the microbial activity controlling material in the method or apparatus for supplying a microbial activity controlling material, for instance, when a material which functions as electron donor which is an energy source of microorganism is used singly, it is possible to control the activity of a kind of microorganism which needs the electron donor among the microorganisms residing in the ambient region around the vessel. When an acidic material or a basic material is used, it is possible to control pH around the vessel so as to control the activities of microorganisms. When an inorganic salt is used, an environment profitable to the maintenance of microbial activity or to the multiplication of the microorganisms can be established by improving the concentration of the inorganic salt at the ambient region around the vessel, and thus the activation of the microorganisms can be accelerated. When an oxygen releasing material is used, it is possible to control the activation of aerobic microorganisms among the microorganisms residing in the ambient region around the vessel by supplying oxygen to the ambient region around the vessel and thus creating an aerobic environment therein. When an oxygen absorbing material is used, it is possible to control the activation of anaerobic microorganisms among the microorganisms residing in the ambient region around the vessel by absorbing oxygen from the ambient region around the vessel and thus creating an anaerobic environment therein.

When two or more kind of the microbial activity controlling materials are filled concurrently in a vessel, or two or more microbial activity controlling material supplying apparatuses each of which is filled with a different microbial activity controlling material are used in combination, it is possible to practice selectively and concurrently any combination of two or more of: the control of activity of microorganism by supplying the electron donor, the control of activity of microorganism by pH control, the acceleration of activation of microorganism by increment of the inorganic salt's concentration, the creation of aerobic environment by supplying oxygen, and the creation of anaerobic environment by absorbing oxygen, so that the control of the activity of microorganism is preformed in combination or synergistically, and thus it becomes possible to control activities of various microorganisms.

The electron donor used as the microbial activity controlling material may be one or more members selected from a group of hydrogen, hydrogen sulfide and organic compounds permeable through the non-porous film.

Further, it is more desirable from the environmental view point and economical view point to use a waste alcohol as the electron donor material. The non-porous film plays a role of "molecular sieve", and the larger the molecular weight of molecule to be permeated becomes, the more difficult the molecule permeates through the film. In addition, depending on the properties of the molecule such as the polarity of the molecule, the permeability of the molecule may be varied. Therefore, when the non-porous film is prepared as a hydrophobic film, for example, represented by polyethylene or polypropylene film, and the electron donor material used contains impurities such as catechin or cyanide which show toxicity to the microorganism, the catechin the size of which is amply large, and the cyanide which shows a high polarity can hardly permeate through the film, and thus, it is possible to permeate a composition of which main ingredient is the electron donor material which is harmless to the microorganism. The reuse of waste alcohol is preferable for the environment because the amount of waste material can be decreased, and also preferable from a view point of the cost reduction of the energy source as utilization of the waste material. For example, in the present invention, it is possible to use the waste alcohol per se, without treating it in advance by distillation and purification, etc., while in the currently known method the waste alcohol is reused after it is subjected to such distillation and purification, etc. Therefore, a large cost down can be expected. More concretely, the waste alcohol, which is produced from a manufacturing process of a food or pharmaceutical product, can be effectively used as an energy source for microorganisms, without providing in advance a removal process such as distillation, for removing the material (catechin, etc.) which shows toxicity to the microorganisms.

Incidentally, the electron donor material can be gently released to the ambient region around the vessel, regardless of its state, namely, being in liquid state or in gaseous state, by bringing the molecules thereof into a gentle permeation through the non-porous film. Therefore, even when in vessel a volatile organic material such as alcohol, benzene, toluene, phenol, etc, is existed in a mixture state of liquid and gaseous phases thereof, the material can be gently released through the whole face of the non-porous film.

As acidic material used as the microbial activity controlling material, for instance, hydrochloric acid, sulfuric acid, nitric acid, and other inorganic acids, acetic acid and other organic acids can be enumerated, but the acidic material is not limited thereto. As basic material, for instance, sodium hydroxide, ammonia, etc., can be enumerated, but the basic material is not limited thereto.

As inorganic salt used as the microbial activity controlling material, for example, ammonium sulfate and other ammonium salts, potassium nitrate and other nitrates, potassium phosphate and other phosphates can be enumerated, but inorganic salt is not limited thereto.

As oxygen releasing material used as the microbial activity controlling material, for instance, oxygen and air are enumerated. In addition, oxygen generating materials such as hydrogen peroxide solution, sodium percarbonate - hydrogen peroxide adduct, calcium peroxide, magnesium peroxide, etc., can be also enumerated. When using the hydrogen peroxide solution, manganese dioxide or potassium permanganate may be used concurrently as an oxygen generating catalyst.

As oxygen absorbing material used as the microbial activity controlling material, for instance, solid reducing agents such as reduced iron, and solution including the reducing agent such as sodium sulfite solution can be enumerated.

Next, as vessel for storing the microbial activity controlling material, any one at least a part of which is composed of a non-porous film is adaptable, more preferably, the one which is wholly composed of the non-porous film is desirable. Using the latter constitution, it is possible to release the microbial activity controlling material throughout the reactor.

As the constitution of the vessel, although one in which the non-porous film is put on a framework is adaptable, but one in which the non-porous film per se is formed as a bag or tube and sealed in the state that the microbial activity controlling material is stored therein is desirable. When using such a constitution, it becomes a form particularly suitable to handling, and by which form the microbial activity controlling material is gently put out at a constant rate automatically during the microbial activity controlling material remains in the vessel. Therefore, there is no need for maintaining the apparatus, and it is possible to make the constitution of the apparatus a simplified one. When the microbial activity controlling material stored in the vessel with a sealed state is consumed, only a simple operation that the consumed one is replaced with a fresh vessel in the shape of bag or tube and in which the microbial activity controlling material is stored in a sealed state is required. The used vessel of a bag or tube shape can be reused as a resource by recycling.

Alternatively, it is also preferable that the vessel has a constitution capable of refilling the microbial activity controlling material. For instance, a supply part for refilling the microbial activity controlling material into the vessel is provided on the vessel. When a liquid microbial activity controlling material is used, it is desirable to provide a tank part, to which the microbial activity controlling material is temporary reserved, in the supply part, and to integrate thus constituted supply part with the vessel. It is also desirable to communicate the vessel with a reservoir tank for the microbial activity controlling material, and if necessary, to provide a supply nozzle by which the microbial activity controlling material can be refilled. In this case, as far as the microbial activity controlling material is reserved in the tank, as the volume of the microbial activity controlling material in the bag (vessel) decreases, the microbial activity controlling material can be added to the vessel from the tank, with the aid of the differential pressure between the tank and the vessel.

In the method or apparatus for supplying a microbial activity controlling material to the microorganisms according to the present invention, as the non-porous film, one of hydrophobic films, hydrophilic films, and amphipathic films, can be used in accordance with the properties of the microbial activity controlling material which fills in the vessel.

Next, in the apparatus for supplying a microbial activity controlling material to the microorganisms according to the present invention, it is preferable to provide a protective member on the surface of the non-porous film in order to protect the non-porous film from wearing and external impacts such as external force. As the protective member, for instance, a nylon net or a non-woven fabric can be used, but it is not limited thereto. When covering a part of or the whole surface of the non-porous film with the protective member, it is possible to protect the non-porous film from the external impact. Further, when the protective member is given a cylindrical or sheath shape with the aid of a rigid member, and the microbial activity controlling material supplying apparatus is inserted in the protective member, it is possible to prevent the microbial activity controlling material supplying apparatus from swelling, and thus possible to make it thinner. Therefore, the installation density of the microbial activity controlling material supplying apparatuses to a treating tank or the like can be enhanced.

Further, when a carrier which is able to immobilize microorganisms is provided on the surface of the non-porous film of the microbial activity controlling material supplying apparatus according to the present invention, it becomes possible to allow the microorganisms to reside onto the non-porous film artificially or naturally, and thereby, to control the activities of the microorganisms efficiently.

Next, in the method for environmental purification according to the present invention, environment polluting materials is removed by placing the microbial activity controlling material supplying apparatus in a decontamination targeting area, supplying the microbial activity controlling material to the ambient region around the microbial activity controlling material supplying apparatus, and thereby, controlling the activity of the microorganisms resided at the ambient region around the microbial activity controlling material supplying apparatus. Therefore, once a simple operation that the microbial activity controlling material supplying apparatus according to the present invention is set to the decontamination targeting area is performed, the microbial activity controlling material is constantly and gently supplied to the decontamination targeting area so as to activate the microorganisms residing in the decontamination targeting area, and thus, it is possible to accomplish the removal of the environment polluting material.

Incidentally, the "decontamination targeting area" used herein means mainly one of soil, ground water, sludge, river and ocean, at which the environment polluting material exists, but it is not limited thereto. Further the "microorganisms resided at the ambient region around the microbial activity controlling material supplying apparatus" is not limited only to the microorganisms which live in the region originally, but also includes microorganisms newly added to the region as well as microorganisms immobilized on the carrier which is provided on the surface of the non-porous film. Furthermore, any facilities where microorganisms effective for a decomposition treatment of the environment polluting material are artificially added, for instance, waste water treating facilities utilizing the capability of the microorganisms for treating the polluting material, are also included.

In the method for environmental purification according to the present invention, when an electron donor is supplied as the microbial activity controlling material and the microorganisms which needs the electron donor among the microorganisms are activated, the environment polluting material can be removed. Namely, the microorganisms which need the electron donor are selectively activated and functionalized from the microorganisms residing in the ambient region around the microbial activity controlling material supplying apparatus.

When the permeating amount of the molecules of electron donor material per unit area in which the molecules of electron donor material is gently released through the non-porous film is regulated to a minimum amount necessitated for keeping the microorganisms alive, it is possible to keep the microorganisms which require the electron donor alive for a long time at the ambient region around the vessel, by supplying the electron donor material to the microorganisms which require the electron donor at a level of not inducing the activation of the microorganisms. Therefore, it is possible to keep microorganisms which are residing under a certain environment alive without causing deaths of the microorganisms. Further, when the electron donor material is supplied at a minimum amount necessitated for keeping the microorganisms alive, it is possible to perform the removal of the environment polluting material efficiently by utilizing co-metabolism of microorganisms.

Separately, when supplying oxygen, it is possible to remove the environment polluting material by activating aerobic microorganisms among the microorganisms resided in the ambient region around the microbial activity controlling material supplying apparatus.

Further, when a microbial activity controlling material supplying apparatus into which an electron donor material is filled is placed as a first apparatus for supplying microbial activity controlling material in the decontamination targeting area, and another microbial activity controlling material supplying apparatus into which an oxygen absorbing material is filled is placed as a second apparatus for supplying microbial activity controlling material at a location where an anaerobic circumstance is formed by absorbing oxygen from the ambient region around the first apparatus, it is possible to perform the removal of the environment polluting material by activating anaerobic microorganisms which require the electron donor material among the microorganisms resided in the ambient region around the first apparatus. Therefore, even at a place which tends to become the aerobic circumstance, such as a place near the Earth's surface, and a place into which ground water containing rich oxygen flows or permeates constantly, it is possible to activate the anaerobic microorganisms.

Alternatively, when a microbial activity controlling material supplying apparatus into which an electron donor material is filled is placed as a first apparatus for supplying microbial activity controlling material in the decontamination targeting area, and another microbial activity controlling material supplying apparatus into which an oxygen releasing material is filled is placed as a second apparatus for supplying microbial activity controlling material at a location where an aerobic circumstance is formed at the ambient region around the first apparatus, it is possible to perform the removal of the environment polluting material by activating aerobic microorganisms which require the electron donor material among the microorganisms resided in the ambient region around the first apparatus.

Separately, when a microbial activity controlling material supplying apparatus into which an electron donor material is filled is placed as a first apparatus for supplying microbial activity controlling material in the decontamination targeting area, and another microbial activity controlling material supplying apparatus into which an oxygen releasing material is filled is placed as a second apparatus for supplying microbial activity controlling material at a location where oxygen is not supplied and which is near the first apparatus, so that the material produced by the activated aerobic microorganisms resided at the ambient region around the second apparatus can be supplied to other microorganisms resided at the ambient region around the first apparatus, it is possible to perform the removal of the environment polluting material by using the microorganisms which require the electron donor material among the microorganisms resided in the ambient region around the first apparatus, and the aerobic microorganisms among the microorganisms resided in the ambient region around the second apparatus.

Further, when a microbial activity controlling material supplying apparatus into which an electron donor material is filled is placed as a first apparatus for supplying microbial activity controlling material in the decontamination targeting area, and another microbial activity controlling material supplying apparatus into which an oxygen releasing material is filled is placed as a second apparatus for supplying microbial activity controlling material at a location where oxygen is not supplied and which is near the first apparatus, so that the material produced by the activated aerobic microorganisms resided at the ambient region around the second apparatus can be supplied to other microorganisms resided at the ambient region around the first apparatus, and still another microbial activity controlling material supplying apparatus into which an oxygen absorbing material is filled is placed as a third apparatus for supplying microbial activity controlling material at a location where oxygen is not supplied from the second apparatus and where the an anaerobic circumstance is formed by absorbing oxygen from the ambient region around the first apparatus, it is possible to perform the removal of the environment polluting material by using anaerobic microorganisms which require the electron donor material among the anaerobic microorganisms resided in the ambient region around the first apparatus, and aerobic microorganisms among the microorganisms resided in the ambient region around the second apparatus.

The microbial activity controlling material supplying apparatus as mentioned above can be utilized on all of facilities or bioreactors in which the supply of microbial activity controlling material to the microorganisms is necessitated. For instance, it is possible to constitute a bioreactor wherein a carrier onto which microorganisms which is effective in the removal of a target component are immobilized is arranged around non-porous film parts of the microbial activity controlling material supplying apparatus. It is also possible to constitute a bioreactor wherein a carrier is made as a bag, and the apparatus of supplying the microbial activity controlling material to the microorganisms is installed in the interior space of the bag.

In such a case, since the microbial activity controlling material which is filled in the vessel of the microbial activity controlling material supplying apparatus permeates through the non-porous film and thus it is gently released to the ambient region around the vessel, the microbial activity controlling material is directly, or after once released in the bag of the carrier, supplied to the microorganisms immobilized on the carrier. That is, it is possible to supply the microbial activity controlling material autonomously, uniformly, and with a gentle sustained-release, while the microbial activity controlling material is installed near the microorganisms.
Therefore, it is possible to handle the microbial activity controlling material supplying apparatus independently each bioreactor, and thus it is convenient to use. Further, since the microbial activity controlling material allows to permeate at a rate which is controlled by the molecular permeability of the non-porous film, there is substantially no possibility that the microbial activity controlling material is consumed wastefully or that the region to be treated is contaminated with the microbial activity controlling material.

Further, the bioreactor according to the present invention may take a constitution that microorganisms are directly carried on the surface of the non-porous film of the vessel in the above mentioned microbial activity controlling material supplying apparatus. In this case, since the microorganisms are immobilized on the surface through which the microbial activity controlling material permeates, almost the whole amount of the supplied microbial activity controlling material is directly supplied to the microorganisms. In such a case, the immobilization of the microorganisms is stimulated by applying a hydrophilic treatment to the surface of the non-porous film so that a polymer gel can cling easily to the surface, or by applying a napping treatment to the surface of the non-porous film so that the microorganisms can cling directly to the surface. In this case, there is no need of preparing a separate vessel to which carrier such as gel is attached.

In the bioreactor according to the present invention, the carrier may be composed of a water absorbent polymer. In this case, when the bioreactor is settled in the atmosphere in order to remove a target component from the atmosphere, the target component can be removed from the atmosphere by dissolving the target component into water which is included in the gel. Incidentally, in the case that the bioreactor is used in the atmosphere, it becomes necessary to supply water at regular time intervals because the immobilized microorganisms may die by dry. When using the water absorbent polymer is used, however, it is possible to lessen the times for the water supplying, or more preferably, it is possible to omit the water supplying when water is adequately supplied by water presented in the atmosphere. Further, when using the water absorbent polymer, the water holding capacity and the water absorbent power can be improved as compared with the case of using a polymer gel. Thus, even when the bioreactor having the water absorbent polymer as the carrier is used at a solid phase or liquid phase region to be treated, such as soil or ground water, it is possible to remove the environment polluting material for a long time while carrying the microorganisms under a good condition, because the water absorbent polymer can absorb water and hold it efficiently. Furthermore, even when the water content of the soil becomes lower due to the degression of rainfall, it is possible to prevent the microorganism from their deaths because the carrier becomes resistant to dry owing to the water holding capacity of the water absorbent polymer.

The bioreactor according to the present invention may be a bioreactor wherein one or more kinds of microorganisms which are effective for removing a target material from a region to be treated, the region being in one of liquid phase, gaseous phase and solid phase, and one or more kinds of microorganisms which can oxidize or reduce the material produced by the former microorganisms are immobilized to a carrier, and a liquid to be treated is subjected to contact with one face of the carrier while a microbial activity controlling material is subjected to contact with the other face of the carrier. The microbial activity controlling material used may be a material which functions as an electron donor which becomes an energy source of the microorganisms.
As examples of such microorganisms, for instance, ammonia-oxidizing bacteria as the microorganism useful for removing the target material, and denitrifying bacteria as the microorganism useful for reducing the material produced by the microorganism useful for removing the target material, are enumerated, respectively. Further, when nitrite-oxidizing bacteria is used as the microorganism useful for oxidizing the material produced by the microorganism useful for removing the target material, it is possible to complete the nitrogen removal, via a route of oxidizing the nitrite ion to the nitrate ion. Therefore, it is possible to convert ammonia, nitrate ion and nitrite ion, which belong to the nitrogen compound, into a harmless nitrogen gas.

As the circumstance into which the bioreactor according to the present invention is utilized, it may be in the atmosphere, in soil, or in water. More concretely, the bioreactor can be used in soil, ground water, sludge, waste water, river, ocean, etc., at which the environment polluting material exists, or used in the atmosphere, etc., in which a harmful gas exists.

### BRIEF DESCRIPTION OF THE DRAWINGS

[Fig. 1] are diagrams showing an example of sealed type which is one embodiment of the microbial activity controlling material supplying apparatus according to the present invention, wherein (A) is a perspective view, and (B) is a longitudinal sectional view.
[Fig. 2] are diagrams showing an example of refilling type which is another embodiment of the microbial activity controlling material supplying apparatus according to the present invention, wherein (A) is a perspective view, and (B) is a longitudinal sectional view.
[Fig. 3] is a schematic diagram showing the general view of the microbial activity controlling material supplying apparatus in the embodiment shown in Fig. 2.
[Fig. 4] is a perspective view of an example of the constitution of a bioreactor utilizing the microbial activity controlling material supplying apparatus according to the present invention.
[Fig. 5] are longitudinal sectional views showing examples of the constitutions of bioreactors utilizing the microbial activity controlling material supplying apparatus according to the present invention, wherein (A) is an example of the sealed type, and (B) is an example of the refilling type, respectively.
[Fig. 6] are graphs showing the results from determination of permeated volumes of organic matters through a polyethylene film, wherein (A) is the results for methanol, and (B) is the results for ethanol, respectively.
[Fig.7] are graphs showing the results of performance elevation when a microbial activity controlling material supplying apparatus in which ethanol was sealed in a polyethylene film was used in an bioreactor wherein an immobilized carrier was used, wherein (A) shows a relation between the ammonia concentration and the course of days, and (B) shows a relation between the nitrite concentration and the course of days
[Fig. 8] is a diagram showing an embodiment where an electron donor supplying apparatus is used in the condition of being buried in soil.
[Fig. 9] is a diagram showing an embodiment where an electron donor supplying apparatus and an oxygen absorbing apparatus are used in the condition of being buried in soil.
[Fig.10] is a diagram showing an embodiment where an electron donor supplying apparatus and an oxygen supplying apparatus are used in the condition of being buried in soil.
[Fig. 11] are diagrams showing an embodiment where a carrier and a protective material are provided in a electron donor supplying apparatus, wherein (A) is a perspective view, and (B) is a longitudinal sectional view.
[Fig. 12] is a graph showing results from determination of permeated volumes of ethanol, acetic acid, lactic acid, and glucose through a polyethylene film.
[Fig. 13] is a graph showing results from determination of permeated volumes of glucose and sucrose through a polyvinyl alcohol film.
[Fig. 14] is a graph showing results from determination of permeated volumes of various ions through a polyethylene film.
[Fig. 15] is a graph showing results from determination of permeated volumes of various ions through a polyvinyl alcohol film.
[Fig. 16] is a graph showing results from determination of pH value at an ambient environment when an acidic material or a basic material is released through a polyethylene film.
[Fig. 17] is a conceptual diagram showing an example of improvement of exhausted soil by using the microbial activity controlling material supplying apparatus according to the present invention.
[Fig. 18] is a conceptual diagram showing an example of treating effluent waste oil by the microbial activity controlling material supplying apparatus according to the present invention.
[Fig. 19] is a diagrams showing another embodiment where an electron donor supplying apparatus and an oxygen supplying apparatus are used in the condition of being buried in soil.

### EXPLANATION OF NUMERALS

1 Microbial activity controlling material supplying apparatus (Electron donor supplying apparatus)
2 Non-porous film
3 Microbial activity controlling material
4 Vessel
4a Microbial activity controlling material reservoir part
5 Supplying part
6 Microbial activity controlling material reservoir tank
7 Supplying nozzle
8 Tube
9 Bioreactor
10 Non-woven fabric
11 Carrier
12 Pocket for microbial activity controlling material supplying apparatus
13 Denitrifying bacteria
16 Protective material
17 Carrier
20 Oxygen supply apparatus
21 Oxygen absorbing material
22 Oxygen
23 Ammonia-oxidizing bacteria
25 Oxygen absorbing apparatus

### BEST MODE FOR CARRYING OUT THE INVENTION

Now, the constitution of the present invention will be described in detail on the basis of embodiments shown in Figures.

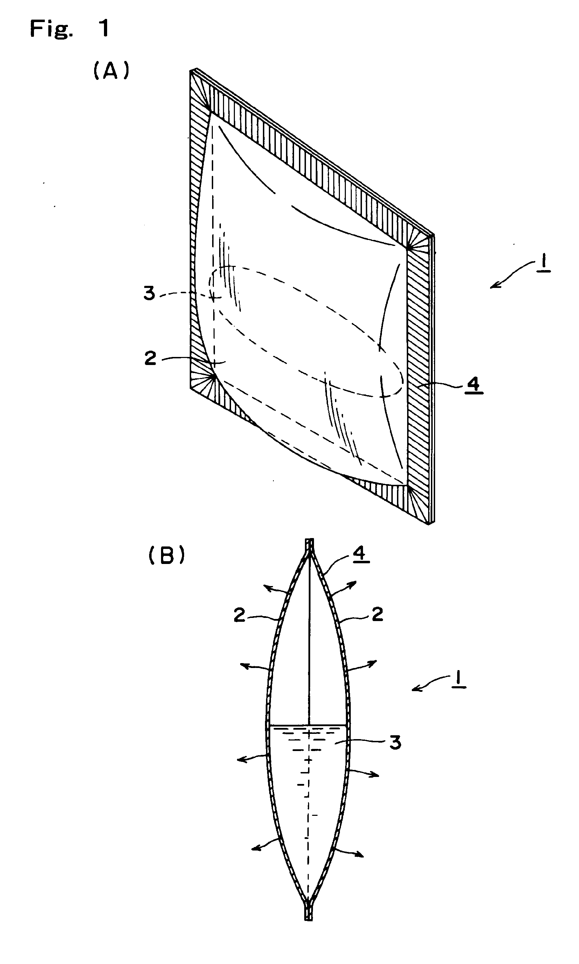

An embodiment of the microbial activity controlling material supplying apparatus according to the present invention is shown in Fig. 1. This microbial activity controlling material supplying apparatus 1 includes a microbial activity controlling material 3, and a vessel 4 which has a sealed structure and at least a part of which is provided with a non-porous film 2, wherein vessel 4 is filled with the microbial activity controlling material 3 and supplies the microbial activity controlling material 3 through the part of the non-porous film 2 of the vessel 4 to ambient places around the vessel 4 at the speed controlled by the molecular permeation performance of the non-porous film 2, and thereby controlling the activities of microorganisms residing around the vessel 4. In this embodiment, the whole part of vessel 4 is composed of the non-porous film 2 which forms a bag, and the periphery parts thereof are welded to each other by heat-seal or adhered to each other with adhesive agent so as to seal the microbial activity controlling material 3 in the vessel. The shape and constitution of the vessel, however, are not particularly limited. For example, the vessel 4 can be formed as a tube or a sheet. Alternatively, the vessel 4 is formed so as to taper off to the point with using a harder material, and is used by putting into the soil. Further, as for the bag-shaped vessel (it is also simply called "bag".), it is not especially limited to the one the whole part of which is composed of the non-porous film, it may be a bag only one side of which comprises the non-porous film, or a bag only a part of one side of which comprises the non-porous film. In the case that the non-porous film is used as a part of the vessel, other parts of the vessel may comprise a rigid frame of metal or plastic, or a film which does not permeates the microbial activity controlling material.

The microbial activity controlling material supplying apparatus according to the present invention can control various microbial activities by releasing gently the microbial activity controlling material 3 which has been filled in the vessel 4. As the microbial activity controlling material 3, electron donor materials, acidic materials, basic materials, inorganic salts, oxygen releasing materials, and oxygen absorbing materials are enumerated. These microbial activity controlling materials may be used singly, or in a combination in which the materials used do not counteract mutually. For example, when using an electron donor material, it is possible to activate microorganisms which need the electron donor among the microorganisms residing in the ambient region around the vessel 4. In this case, the microbial activity controlling material supplying apparatus can be also called an electron donor supplying apparatus. When using an acidic material or a basic material, it is possible to control pH to a desired value around the vessel 4 so as to prepare an environment profitable to a microorganism to be activated. When using an inorganic salt, it is possible to make the concentration of inorganic salt necessitated for growth and activity of microorganism, for instance, the concentration of inorganic salt which involves nitrogen and phosphorus, risen, so as to prepare an environment profitable to microorganisms residing in the ambient region around the vessel 4. When using an oxygen releasing material, it is possible to supply oxygen around the vessel 4 and thus to form an aerobic circumstance, so as to activate aerobic microorganisms among the microorganisms residing in the ambient region around the vessel 4. Since the aerobic circumstance thus prepared is not preferable to anaerobic microorganisms, it is also possible to inactivate the anaerobic microorganisms. When using an oxygen absorbing material, it is possible to absorb oxygen around the vessel 4 and thus to form an anaerobic circumstance, so as to activate anaerobic microorganisms among the microorganisms residing in the ambient region around the vessel 4. Since the anaerobic circumstance thus prepared is not preferable to aerobic microorganisms, it is also possible to inactivate the aerobic microorganisms.

When using two or more of microbial activity controlling materials in combination, it is possible to enhance the microbial activity controlling effect by synergy of individual microbial activity controlling effects of microbial activity controlling materials used. For instance, when an electron donor material and an oxygen releasing material are used in combination, it is possible to activate aerobic microorganisms which need the electron donor even under anaerobic circumstance. When an acidic material or a basic material is further added to the above combination, it is possible to provide an environment which pH is preferable to aerobic microorganisms which need the electron donor. When an inorganic salt is further added to the above combination, it is possible to provide an environment where aerobic microorganisms which need the electron donor are easy to multiply, and thus, possible to multiply the microorganisms to be activated, and to improve the activation efficiency.

Incidentally, in the case that two or more of such materials are used in combination, it is possible to fill these materials in a mixture state into a vessel 4. Alternatively, the gentle release of these materials may be attained by partitioning the vessel 4, and filling the materials to thus formed respective partitions. Otherwise, these materials may be filled in the respective vessels, and the vessels are arranged in mutually neighboring condition.

The non-porous film 2 used in the microbial activity controlling material supplying apparatus of the present invention is what the microbial activity controlling material 3 is gently released by allowing molecules of the material 3 to permeate it little by little. This non-porous film 2 can control permeation rate of the microbial activity controlling material molecules per unit area of the film, by adjusting the material kind or thickness of the film, molecular weight or characteristic of the microbial activity controlling material 3, temperature, or concentration of the microbial activity controlling material. For instance, when the concentration of the microbial activity controlling material used is heightened, it is possible to increase the permeation rate of the microbial activity controlling material even with the same vessel. Thus, by selecting the concentration of the microbial activity controlling material depending on the desired permeation rate, the activity of microorganism can be controlled. Separately, by varying the surface area of the non-porous film, the releasing area for the microbial activity controlling material can be regulated. Therefore, by contacting the non-porous film 2 to a part of the region to be treated, the releasing area can be varied according to the contacting area between the region to be treated and the non-porous film. According to our, the inventors', experiment for the polyethylene film, it has been confirmed that the molecular permeation rate per unit area can be varied depending to the thickness of the film among the films of the same material. Thus, by choosing the thickness of the non-porous film depending on the supply amount necessitated for controlling the microorganism's activity, the microbial activity controlling material can be supplied at a necessitated rate and with a necessitated amount. In this situation, the microbial activity controlling material supplying apparatus releases at a gentle speed controlled by the molecular permeation ability of the non-porous film. Thus, the microbial activity controlling material can be supplied with its diluted condition in which concentration does not affect the survival of microorganisms residing the ambient region around the vessel, even when undiluted alcohol is filled as the microbial activity controlling material in the vessel, wherein such undiluted alcohol per se may cause the microorganisms to die when it is supplied in its intact form.

The non-porous film 2 can also vary its molecular permeation rate depending to the density and structure of molecules which constitute the film. Here, the case of polyethylene will be exemplified. When the high density polyethylene (density: not less than 942 kg/m³), classified in accordance with JIS K 6922-2, is used, the permeation rate of the microbial activity controlling material supplying apparatus out of the film is decreased, as compared with the case of the low density polyethylene (density: not less than 910 kg/m³ and less than 930 kg/m³) is used. Therefore, in accordance with the desirability of microbial activity controlling material supplying apparatus, the supplying amount of the microbial activity controlling material can be regulated by the valance between thickness and density of the non-porous film 2.
Further, molecular structure of polyethylene chain in the film can be modified by a certain way such as orientation treatment. Therefore, by adopting such treatment to vary the film density or molecular structure of the desired film material, the supplying amount of the microbial activity controlling material can be controlled.

As the non-porous film 2, one of hydrophobic films, hydrophilic films, and amphipathic films can be used in accordance with the properties of the microbial activity controlling material which fills in the vessel. As hydrophobic film, for instance, olefin type films such as polyethylene, polypropylene, etc., are enumerated. As hydrophilic film, for instance, films having hydrophilic groups in its molecular structure such as polyester, nylon (polyamide), polyvinyl alcohol, vinylon, cellophane, polyglutamic acid, etc., are enumerated. As amphipathic film, for instance, ethylene - vinyl alcohol copolymer (EVOH), namely, a copolymer film which has both hydrophobic polyethylene structure and hydrophilic polyvinyl alcohol structure, is enumerated. With respect to the amphipathic film, it is possible to enhance one of the hydrophobic moiety and hydrophilic moiety by changing the containing ratio of hydrophobic polyethylene and hydrophilic polyvinyl alcohol. In addition, although the permeation properties of the following films are inferior to those of the above enumerated non-porous film, when a extremely slow controlled release is required, vapor-liquid type films, namely, films of which permeation property varies depending to the states of hydrophilic groups or polar groups in their molecular structure, such as polyvinylidene chloride, polycarbonate, ethylene - acrylic acid copolymer, polyethylene terephthalate mixture type, etc., may be enumerated.

When using a hydrophobic film such as polyethylene, polypropylene, etc., which is a material of low-cost, good durability, good chemical resistance, and stable, it becomes possible to permeate volatile organic materials such as benzene, toluene, etc., which are hydrophobic materials having substantially no hydrophilic group in their molecular structures. Further, according to our, the inventors', experiments, it was confirmed that some of materials which has hydrophilic groups in their molecular structures, such as methanol, ethanol, and other alcohols, acetic acid, etc., can permeate the hydrophobic film. On the other hand, materials having high water-solubility such as ions and saccharides can not permeate the hydrophobic film. In such a case, when a hydrophilic film such as polyvinyl alcohol film is used, it becomes possible to allow the materials having high water-solubility to permeate the film. Since methanol, ethanol, and acetic acid can be dissolved to water, they can permeate the polyvinyl alcohol film. Incidentally, according to our, the inventors', experiments, it was confirmed that acetic acid which is an acidic material, and ammonia gas which is a basic material can permeate polyethylene film. Further, it was also confirmed that hydrochloric acid which is a strong acid, and sodium hydroxide which is a strong base can permeate the polyvinyl alcohol film with ease, while they hardly permeate polyethylene film. Such phenomena are due to the fact strong acids and strong bases are dissociated completely in water so as to become water-soluble ions. Thus, when using hydrophobic film (s) such as polyethylene film, or hydrophilic film (s) such as polyvinyl alcohol film, as the characteristic of acidic material or basic material which is desired to permeate, it becomes possible to control pH of ambient region around the vessel.

Further, when using ethylene - vinyl alcohol copolymer (EVOH) which has both hydrophilic and hydrophobic properties, it becomes possible to permeate hydrophilic material(s) and hydrophobic material(s) concurrently. It is also possible to control the permeation rates of materials by the ratio of ethylene and vinyl alcohol in the copolymer. Namely, when the ethylene moiety is increased, it becomes possible to enhance the permeation rate of the hydrophobic material, while when the vinyl alcohol moiety is increased, it becomes possible to enhance the permeation rate of the hydrophilic material.

Incidentally, polyethylene or polypropylene can separate certainly the field for activities of a certain microorganism such as water region, in-soil environment, or atmospheric environment, from other regions. Further, they have proper material permeability, and proper heat reversible, as well as good flexibility and workability. In addition, polyethylene and polypropylene are low cost and easy available. Thus, the adoption of polyethylene or polypropylene as the hydrophobic film may bring great advantages in view of cost and properties.

As the electron donor material used as the microbial activity controlling material 3, any material which is the electron donor material necessitated by the interest microorganism and is not toxic to this microorganism, and does not show a corrosive action to the non-porous film 2, and also has a molecular weight and properties capable of permeating the non-porous film 2, can be selected appropriately. The state of the electron donor material used may be gaseous or liquid. As the gaseous material, for instance, hydrogen, hydrogen sulfide, or organic compounds such as methane, ethane, etc., are enumerated. As the liquid material, volatile organic material such as methanol, ethanol, propanol, etc., are enumerated. Further, for some microorganisms, volatile organic material such as benzene, toluene, phenol, etc., may be usable. However, the electron donor material is not limited to the mentioned materials. Further, the electron donor material used does not necessarily consist of one member, but may be a mixture state of two or more of hydrogen, hydrogen sulfide and organic compounds, for supplying the electron donor materials to the interest microorganism. For instance, since hydrogen and hydrogen sulfide are gaseous materials and are hydrophobic, they can use in combination with one or more hydrophobic organic compounds, and can be supplied by permeation through a hydrophobic film or an amphipathic film. Otherwise, as the electron donor materials, two or more of hydrophilic organic compounds can be used in combination, and can be supplied by permeation through a hydrophilic film or an amphipathic film. Incidentally, according to our, the inventors', experiments, it was confirmed that acetic acid permeates a polyethylene film of 0.05 mm in thickness. It was also confirmed that lactic acid permeate a polyethylene film of 0.01 mm in thickness. It was also confirmed that glucose and sucrose permeate a polyvinyl alcohol film of 0.025 mm in thickness. Therefore, when using ethylene - vinyl alcohol copolymer film which has both hydrophilic and hydrophobic properties, it becomes possible to use acetic acid or other carboxylic acids, glucose, sucrose or other saccharides, and lactic acid as electron donor materials.

When alcohol is used as the electron donor material, it can be used in its undiluted form. According to the present invention, since the electron donor material is supplied with a gentle rate to microorganisms, even when the alcohol is used in its undiluted form, the alcohol can be diluted on its supply to the microorganisms. Thus, there is no fear that the microorganism becomes dying. Of course, the alcohol is not necessarily used in its undiluted form. Even when the alcohol is used after diluted with water, or the alcohol includes some impurities, only molecules of alcohol can permeate the non-porous film, and thus, are supplied gently to the microorganisms.

The permeation of the electron donor material through the non-porous film 2 is performed when molecules of the material dissolve into the film, then the dissolved molecules diffuse interior of the film, and finally the molecules reach the opposite surface of the film. Therefore, materials, such as catechin, which have an amply large molecular weight incapable of dissolving into the film, hardly permeate the non-porous film. Since the polyethylene and polypropylene or the like are films which have substantially no functional group having affinity to water, and they are low polarity as well as high hydrophobicity, the water molecules which are polar molecules are difficult to be dissolved into such films. In addition, high polarity materials, such as cyan compounds, which are easily dissolved to water, also hardly permeate the films. Furthermore, since intermolecular hydrogen bonds of water are strong, under the condition of a normal temperature, substantially no water permeates the films. Thus, the non-porous film 2 plays a role of "molecular sieve", through which water, and impurities such as cyan compounds which have high polarity and catechin which has a large molecular weight are hardly possible to permeate, while desired electron donor material can permeate as main ingredient. Therefore, when as non-porous film a hydrophobic film, represented by polyethylene and polypropylene, etc., is used, it becomes possible to use waste alcohol which includes some impurities, for instance, antibiotic molecules which show toxicity to the microorganism interested. Further, regardless the state of the electron donor material in the vessel, namely, gaseous, liquid, or steam (produced by volatilization of a volatile organic material), the material is released as its molecular condition to outside of the vessel. Namely, the electron donor material is released gently in gaseous condition in which molecules are not cohesive against their attracting forces among molecules, in contrast to those in liquid. Thus, the non-porous film 2 can be also represented as gas permeation film. Further, it is possible to release the electron donor material gently in its molecular condition to outside of the vessel, not only in the case that the environment outside the vessel is in gaseous phase (atmosphere, etc.), but also in the case that the environment outside the vessel is in liquid phase (waste water, ground water, etc.).

The permeation of inorganic salts through the non-porous film 2 is performed when inorganic ions dissolve into the film, then the dissolved ions diffuse interior of the film, and finally the ions reach the opposite surface of the film. For instance, when an aqueous solution of sulfate, nitrate, phosphate, ammonium salt, or the like, is filled in the vessel 4 which comprises a hydrophilic non-porous film such as polyvinyl alcohol, sulfate ions, nitrate ions, phosphate ions, or ammonium ions dissolve into the non-porous film, then diffuse interior of the film, and finally reach the opposite surface of the film so that the ions are supplied out of the film.

The permeation of acidic material or basic material through the non-porous film 2 is performed when molecules of the acidic material or basic material dissolve into the film, then the dissolved molecules diffuse interior of the film, and finally the molecules reach the opposite surface of the film. For instance, when acetic acid which is an acidic material, or ammonia gas which is a basic material is filled in the vessel 4 which comprises a hydrophobic non-porous film such as polyethylene or polypropylene, acetic acid molecules, or ammonia molecules dissolve into the non-porous film, then diffuse interior of the film, and finally reach the opposite surface of the film so that the acidic material or the basic material is supplied out of the film, and thus pH of ambient region around the vessel can be varied to form an environment which is fitting for the activity of the microorganism interested. Alternatively, when hydrochloric acid which is an acidic material, or sodium hydroxide which is a basic material is filled in the vessel 4 which comprises a hydrophilic non-porous film such as polyvinyl alcohol, hydrogen ions, or hydroxide ions dissolve into the non-porous film, then diffuse interior of the film, and finally reach the opposite surface of the film so that the acidic material or the basic material is supplied out of the film, and thus pH of ambient region around the vessel can be varied to form an environment which is fitting for the activity of the microorganism interested. In addition, when ethylene - vinyl alcohol copolymer film is used, it is possible to supply acidic aqueous solution such as hydrochloric acid or acetic acid, or basic aqueous solution such as ammonia or sodium hydroxide, out of the film, so as to rise or lower pH of ambient region around the vessel, and to form an environment which is fitting for the activity of the microorganism interested.

Next, the permeation mechanism of ion through the polyvinyl alcohol film will be explained in detail. In dried state, hydrogen bonds are being formed interior of polyvinyl alcohol film. Thus, the film takes a structure in which the fluctuations due to thermal oscillation of molecular chains hardly occur, and gaps between the molecular chains are narrow. Therefore, molecular hardly permeate the film. However, once water molecule enters between the molecular chains and the hydrogen bonds are broken, the film structure changes to that the fluctuations due to thermal oscillation of molecular chains are easy to occur, the water molecule invades more interior of the film. As the result, a channel for water molecule is formed. The molecule being in state of ion can diffuse through this channel for water molecule.

Next, with respect to the oxygen permeation through the non-porous film 2 in the case of using an oxygen releasing material or an oxygen adsorbing material, in the hydrophobic film, typically, polyethylene film, oxygen diffuses through a gap between molecular chains of polyethylene film. In this case, when the polyethylene film is a low density PE film which has a structure in which gaps between the molecular chains are loose and the fluctuations due to thermal oscillation of molecular chains are easy to occurs, the oxygen permeability is relatively high. Separately, in the hydrophilic film, typically, polyvinyl alcohol film, a channel for water molecule is formed by invasion of water molecule as mentioned above. Since the oxygen diffusion rate in water is higher than that in polyethylene film, the oxygen permeability is enhanced depending to the degree of size of the channel for water molecule in the interior of polyvinyl alcohol film.

Incidentally, the non-porous film transmits the microbial activity controlling material 3 by dissolving the material into the film, thus it does not control the kind and amount of the microbial activity controlling material by size and number of pores, in contrast to the case of porous film. Therefore, there is no fear of causing a problem of clogging of pores on a long-duration using, and no need of back washing. Thus, it is possible to use for a long time without maintenance, and to reduce the running cost.

It is preferable that the property of the non-porous film 2 is decided in accordance with the property of the microbial activity controlling material 3 to be used. For instance, when as the non-porous film 2 a hydrophobic one is adopted, it tends to allow molecules having hydrophobic group(s) such as carbon chain to permeate. On the other hand, when as the non-porous film 2 a hydrophilic one is adopted, it tends to allow molecules having hydrophilic group(s) and water soluble molecules to permeate. Thus, in accordance with the property of the microbial activity controlling material 3 to be used, the property of the non-porous film 2 may be determined. Further, when giving polarity to the interior of the film and thus tightening the linkages between the molecular chains which constitute the film and lessening the gap between the molecular chains, it becomes possible to control the molecular permeation property. For instance, a film, such as film of polyvinylidene chloride, where some of hydrogen atoms are substituted by chlorine atoms, and thus constituted by polar molecules, has narrow gaps between the molecular chains, as compared with those of polyethylene. Therefore, the molecular permeation property thereof becomes low. Further, as a film capable of possessing both properties, it is possible to connect hydrophobic films with hydrophilic films, and to control the permeability of the microbial activity controlling material 3 by using it.

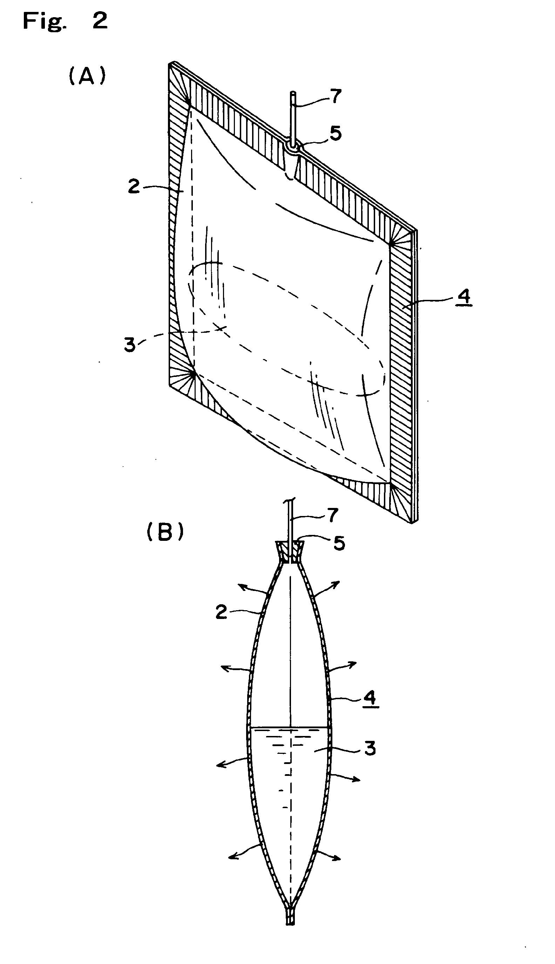

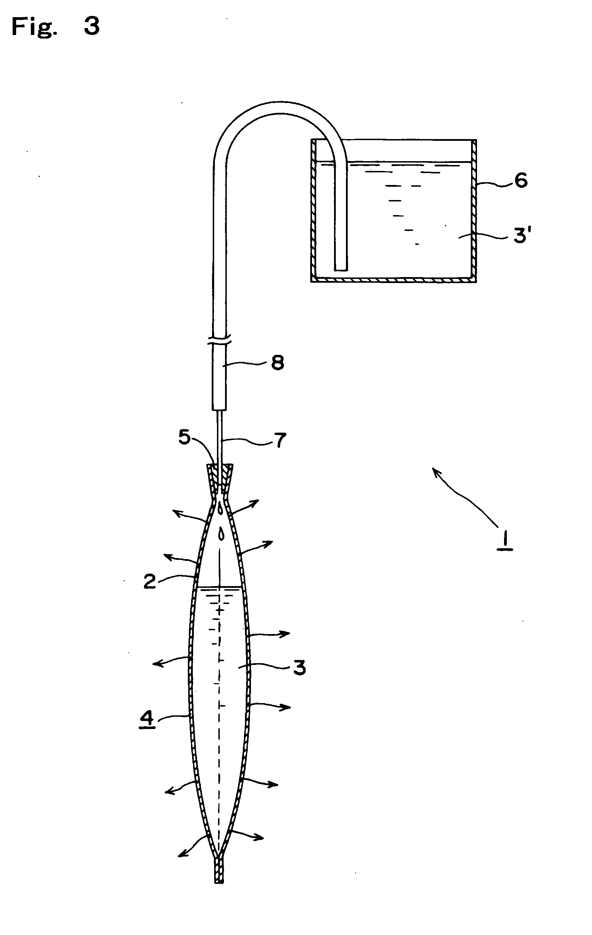

Next, another embodiment is shown in Fig. 2 and Fig. 3. In the microbial activity controlling material supplying apparatus of this embodiment, instead of adapting a completely sealed independent form as the vessel 4 of the non-porous film 2, a vessel is formed into a bag form which is sealed so as to be equipped with a means for introducing the microbial activity controlling material 3, Thus, in this embodiment, it is possible to refill the microbial activity controlling material from the outside of vessel.

The mechanism for refilling the microbial activity controlling material 3 may be a constitution wherein a supply part 5 for introducing the microbial activity controlling material 3 is provided at a portion of the peripheral edge of the vessel 4, and a nozzle or pipe 7 is attached to the supply part 5, or may be a nozzle or pipe 7 which is integrally formed with the vessel in advance. In the microbial activity controlling material supplying apparatus 1 shown in Fig.3, a supply nozzle 7 which is detachably attached to a supply part 5 provided a portion of the peripheral edge of the vessel 4, or a supply nozzle 7 which is integrally formed with the vessel 4, is connected to a tank 6 which reserves a liquid microbial activity controlling material, for instance, alcohol, such as methanol or ethanol, which is a electron donor material, via a tube 8 or the like. Thereby, it becomes possible to refill the microbial activity controlling material 3 on demand. In this constitution, because the tank 6 and the vessel 4 is connected via the tube 8, when the microbial activity controlling material 3 in the vessel 4 is decreased, the microbial activity controlling material 3' can be supplied from the tank 6 to the vessel 4, by virtue of the siphon action, i.e., by virtue of the differential pressure of both ends of the tube 8, as long as the microbial activity controlling material 3' is reserved in the tank 6. Because the vessel is equipped with the supply part5 or the nozzle 7, the vessel is not a sealed structure in the strict sense. However, as long as the condition that the interior of the supply nozzle 7 is filled with the microbial activity controlling material 3' supplied from the tank is maintained, the interior of the vessel holds in a substantially sealed condition, because the liquid surface functions as sealer. Therefore, the liquid or gasified microbial activity controlling material 3 can not leak out from the vessel 4 through the supply part 5 or the nozzle 7.

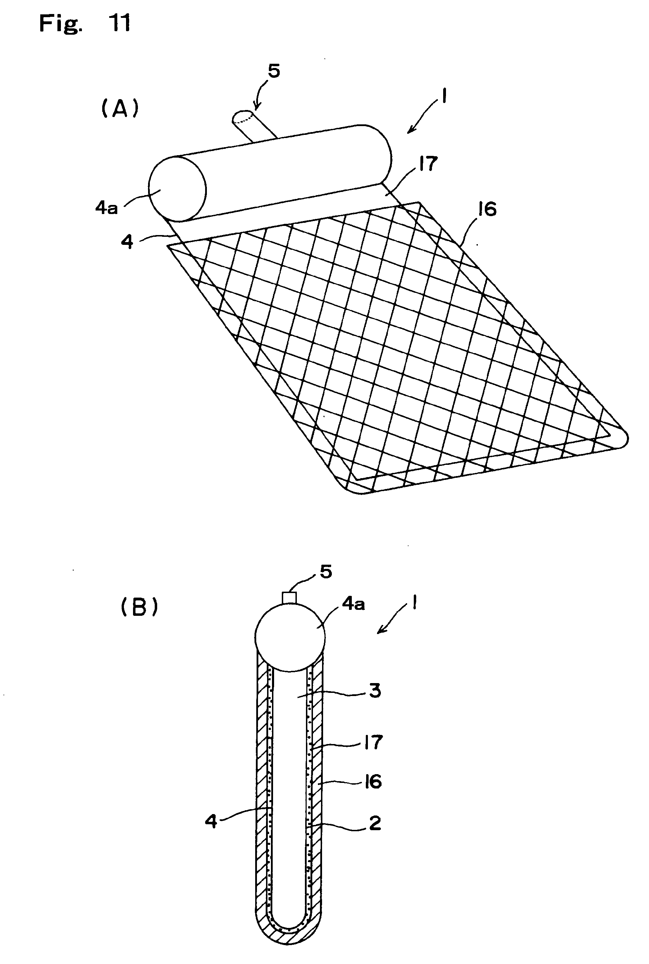

Here, as shown in Fig. 11, it is preferable that a carrier 17 which can immobilize microorganisms is provided on the surface of the bag 4. When equipping the carrier capable of immobilizing microorganisms, it becomes possible to fix the microorganisms artificially, or to allow the microorganisms presenting in every various environments such as in water, in soil and in atmosphere to contact the carrier and then to multiply and colonize on the carrier. As the carrier 17, any one which capable of immobilizing the microorganisms may be usable, for instance, natural polymers such as collagen, fibrin, albumin, casein, cellulose fiber, cellulose triacetate, agar, calcium alginate, carrageenan, agarose, etc.; synthetic polymers such as polyacryl amide, poly-2-hydroxy ethyl methacrylic acid, polyvinyl chloride, Y-methyl polyglutamic acid, polystyrene, polyvinyl pyrrolidone, polydimethyl acryl amide, polyurethane, photo-cross-linkable resins (polyvinyl alcohol derivatives, polyethylene glycol derivatives, polypropylene glycol derivatives, polybutadiene derivatives, etc.); and any mixtures thereof; as well as water absorbent polymers, are enumerated. As the water absorbent polymer, although any known ones may be usable, concretely, for example, polyacrylic acid, polyasparaginic acid, polyglutamic acid, and modified products thereof, and modified product of polyethylene glycol are enumerated. Incidentally, the "modified product" used herein is the product in which a polymer having ionic groups was cross-linked to a part of the aforementioned polymer. When the carrier is provided on the surface of the bag-shaped vessel, the microorganisms immobilized on the carrier are hardly lost out, even at a place where the flow rate of the ground water is constantly high, for example, moisture-rich land such as paddy field, and land under a climate of high temperature and high humidity; and at a place where the flow rate of the ground water rises temporary, for example, due to frequent rainfalls in rainy season. Further, in water environment, it is possible to immobilize the microorganisms on the carrier without diffusing the microorganisms. Therefore, it is possible to maintain the activity of the specific microorganism for a long time under every various environments, and also to control it easily.

The carrier 17 may stick on the surface of the non-porous film via an adhesion agent, or may be integrated with the bag by heat-sealing the carrier concurrently with the heat-sealing of the peripheral edge of bag. The carrier may be provided throughout the whole surface area of the non-porous film, or may be provided at a part of the surface area of the non-porous film. Further, giving hollows appropriately on the carrier, exhaust ports for releasing gas produced by treatment with the microorganisms such as nitrogen gas may be provided. When a water absorbent polymer is used, the water holding ability and the water absorbent ability of the carrier becomes high, as compared with that when a polymer gel is used. Therefore, by absorbing effectively water in the ambient environment and holding it stably, the carrier can keep an environment suitable for living of the microorganism for a long time.

In addition, as shown in Fig. 11, it is preferable to provide a protective member 16 on the surface of the vessel 4. When the protective member 16 is provided, it becomes to protect the non-porous film from wearing by external impact, etc. Further, the protective member 16 also functions as support member for the vessel, and thus it can prevent the vessel 4 from causing tear-off or breakage due to the weight of the microbial activity controlling material 4 filled in the vessel 4. As the protective member 16, the one which does not block the controlled release of the microbial activity controlling material 3 from the non-porous film 2 should be used. In the case of using a highly gas permeable material such as non-woven fabric, even if the protective member covers the whole surface of vessel 4, the controlled release of the microbial activity controlling material 3 is not blocked by the protective member. In the case of using a material which may block the controlled release of the microbial activity controlling material 3, such as metal or resin, by forming it as a net, or by providing numeral minute pores onto it, the controlled release of the microbial activity controlling material 3 can be ensured. Further, when the protective member 16 is given a cylindrical or sheath shape with the aid of a rigid member, it is possible to prevent the bag-shaped vessel resided inside the protective member from swelling with the microbial activity controlling material, and thus possible to fabricate the microbial activity controlling material supplying apparatus more compact. In this case, it become possible to prevent the swelling of the bag-shaped vessel to make the thickness of the bag thinner by restricting the thickness, to enhance the installation density of the microbial activity controlling material supplying apparatuses to a treating tank or the like, and to protect the bag from external impacts.

In Fig. 11, although the protective member 16 is stacked on the surface of the carrier 17, otherwise, the carrier 17 may be stacked outside the protective member 16, or the protective member 16 may use singly or the carrier 17 for immobilizing the microorganisms may use singly. Regardless the arrangement of them, the functions of the carrier 17 and the protective member 16 can be exhibited. Incidentally, the carrier 17 for immobilizing the microorganisms can also function as a protective member, thus can protect the non-porous film from wearing by external impact, etc. Further, by using a polymer, such as photo-cross-linkable resin, which own strength per se is high, and by giving a cylindrical or sheath shape, while, by designing as carrier capable of immobilizing the microorganisms, it is possible to provide a sheath body which function both as the protective member and as the carrier.

Incidentally, the carrier 17 is not limited to above mentioned examples. For example, the carrier may be prepared by subjecting non-woven fabric to a napping treatment, and the microorganisms may be immobilized to the napped portions. Alternatively, it is also possible to apply to the surface of the non-porous film 2 the napping treatment, and to immobilize the microorganisms to the napped portions of the non-porous film 2.

Since the microbial activity controlling material supplying apparatus 1 constituted as above can release the microbial activity controlling material gently to ambient region around the bag 4, once the microbial activity controlling material supplying apparatus is set to an environment where the control of the microorganisms activity is required, regardless the set place is in-water, in-soil, or in-atmosphere, activities of the various microorganisms residing in nature or activities of the microorganisms immobilized in advance with using a carrier or others can be controlled, thus it is possible to utilize the microorganisms. Therefore, for instance, once a simple operation that the bag is thrown into a waste water treating tank is performed, electron donor material(s) 3 is supplied to the microorganisms which need the electron donor, and the microorganisms are activated, thus it is possible to accomplish the waste water treatment. In the case that the waste water treating tank is an environment not preferable to the microorganisms which need the electron donor, for instance, in the case that pH is not preferable to the microorganisms, by supplying basic material(s) or an acidic material(s) from the bag 4, an environment having a desirable pH can be prepared. Further, in the case that the concentration of inorganic salt(s) necessitated for multiplying the microorganisms in the waste water treating tank is low, by supplying inorganic salt(s) from the bag 4, an environment desirable for multiplication can be prepared.
Furthermore, by supplying oxygen from the bag 4 to the waste water treating tank, or by absorbing oxygen from the waste water treating tank to the bag 4, an environment preferable for functionalizing the microorganisms can be prepared. Therefore, it becomes possible to perform the waste water treatment using microorganisms with a low cost, briefly, and effectively. In the case that the microbial activity controlling material is set in atmosphere, in soil, in river or in ocean, the environment for activating selectively the microorganisms which can degrade the targeted environment polluting material can be formed similarly, and thus the environment polluting material can be removed.

Now, the environmental purification method for removing the environment polluting material by placing a microbial activity controlling material supplying apparatus 1 in a decontamination targeting area, supplying the microbial activity controlling material 3 to the ambient region around the microbial activity controlling material supplying apparatus 1, and thereby, controlling the activity of the microorganisms resided at the ambient region around the microbial activity controlling material supplying apparatus will be explained in detail by showing concrete examples.

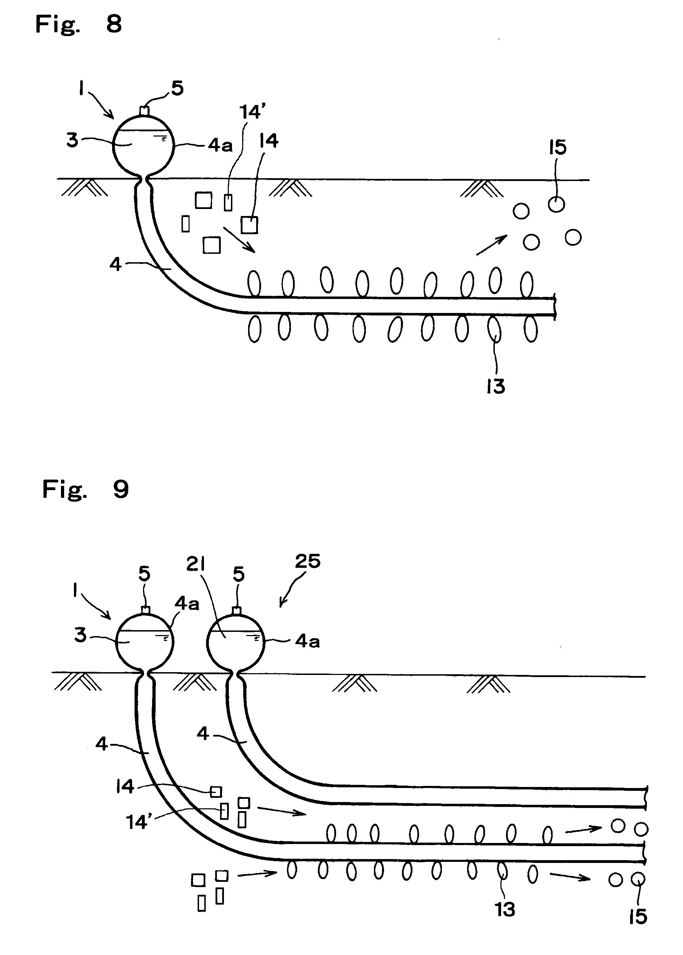

First, as an example of the environmental purification method, a method for removing ammonia, nitrite ion, and nitrate ion, by burying a microbial activity controlling material supplying apparatus 1 in which as the microbial activity controlling material 3 an electron donor material is filled in the vessel 4(hereinafter, the apparatus is referred as "electron donor supplying apparatus 1"), in soil will be explained with referring to Figs. 8 - 10.

The method for removing nitrite ion and nitrate ion from the decontamination targeting area, by using the electron donor supplying apparatus 1 of the present invention will be described with referring to Fig. 8. The electron donor supplying apparatus 1 comprises a vessel which is formed in the bag-shape and is made of non-porous film, for instance a polyethylene bag 4 and an electron donor material 3 which filled in the bag, for instance, alcohol; wherein at the upper area of bag 4 a tank-like reserve part 4a for reserving some extra amount of the electron donor material 3 is provided as being integrated with the bag 4, and wherein a supply part 5 for refilling the electron donor material 3 is further provided at the reserve part 4a. Then, the electron donor material 3 is filled into the bag 4, and the electron donor supplying apparatus is buried in soil in the condition that the reserve part 4a and the supply part 5 are hold above ground and the all other parts are embedded into soil. With monitoring the amount of the electron donor material 3 remained in the aboveground reserved part 4a, the electron donor material 3 will be added at discretion or at predetermined intervals. From the bag 4 embedded in soil, the electron donor material 3 as an electron donor is released out gently through the non-porous film 2, and thus the microorganisms residing at the ambient region around the bag, for example, denitrifying bacteria 13, can be activated. Thus, nitrate ions 14 and nitrite ions 14', which have been accumulated in soil on account of excessive fertilizations, around the bag 4 are converted effectively into harmless nitrogen gas 15. Namely, by burying the electron donor supplying apparatus of the present invention, the microorganisms need the electron donor among the microorganisms residing in the soil of the ambient region around the apparatus can be selectively activated and functionalized. With respect to the microorganisms, of course, it is possible to utilize ones residing in the soil. It is also possible to use microorganisms which was selectively cultured in advance and which has been immobilized on the carrier, when they exists on the surface of bag 4, or they are separately buried into soil. Further, when a vessel 4 which is formed in a tube with tapering off to the point is inserted near the roots of the field crop, it becomes possible to prevent the decreased growth due to the excessive supply of chemical fertilizers. Incidentally, it is preferable that the aboveground reserve part 4a is coated with a film capable of forming a barrier layer which prevents the gentle release of the electron donor material 3.

As the soil deepens, the environment of soil tends to form an anaerobic condition where the aerobic microorganisms can not function. Thus, by using a microbial activity controlling material supplying apparatus in which an oxygen releasing material 22 is filled (hereinafter, the apparatus is referred as "oxygen supplying apparatus 20"), oxygen can be supplied, and the aerobic resting microorganisms or artificially allocated microorganisms can be functionalized. As the oxygen releasing material 22, it is possible to use air or oxygen and to fill them in vessel. Otherwise, the controlled release of oxygen to the ambient region around the vessel can be accomplished by adding an oxygen generating catalyst such as manganese dioxide (MnO₂), potassium permanganate (KMnO₄), etc., in the vessel, providing the supply part 5 in vessel, supplying hydrogen peroxide into the vessel, and thus generating oxygen within the vessel. Separately, without using the oxygen generating catalyst, the controlled release of oxygen to the ambient region around the vessel can be accomplished by supplying only hydrogen peroxide into the vessel. Incidentally, the concentration of the hydrogen peroxide supplied into the vessel becomes lower as the generation of oxygen proceeds, and at the end the hydrogen peroxide is changed to water, and it can generate oxygen no longer. In this case, it is possible to add hydrogen peroxide into the vessel so as to be mixed it with the residual water and allow the oxygen generation again. Otherwise, after the residual water is drawn out, the hydrogen peroxide can be added to the vessel. Incidentally, oxygen may be generated by using a compound other than the hydrogen peroxide, such as sodium percarbonate - hydrogen peroxide adduct, calcium peroxide, magnesium peroxide, etc.

Further, in another approach, while hydrogen, which is obtained by electrolyzing water or sulfuric acid aqueous solution, is introduced into the electron donor supplying apparatus 1, oxygen can be introduced to the oxygen supplying apparatus 20.

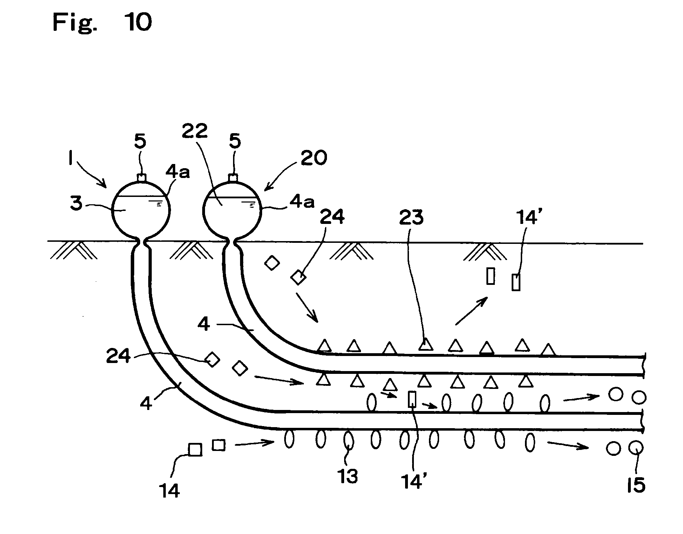

As shown in Fig. 10, when the oxygen supplying apparatus 20 is buried in soil along with the electron donor supplying apparatus 1 and thus oxygen is also supplied, it becomes possible to activate ammonia-oxidizing bacteria 23 which are aerobic microorganisms even under a circumstance where the microorganisms can not active inherently. As a matter of course, at a relatively shallow depth's place where the oxygen exists in a certain contents, it is possible to accelerate the activation of the aerobic microorganisms. Therefore, a series system for making ammonia, nitrate ion and nitrite ion harmless, in which the ammonia 24 in soil is converted to the nitrite ions 14' by the ammonia-oxidizing bacteria 23, then the nitrite ions 14' created by ammonia-oxidizing bacteria, as well as the nitrate ions 14 and the nitrite ions 14' existing in the soil are removed and converted to nitrogen gas 15 by the denitrifying bacteria 13, is formed in soil. Further, by supplying oxygen 22, it becomes possible to activate methane-oxidizing bacteria (not shown). Although there is some possibility that the soil at decontamination targeting area includes nitrate ions 14, nitrite ions 14' and ammonia 24 which originate from chemical fertilizers or farm animals' waste and urine, when supplying oxygen 22 as mentioned above, it becomes possible to oxidize ammonia 24 to nitrite ions 14' by the ammonia-oxidizing bacteria 23; and further when being capable of utilizing methane gas produced from the farm animals' waste and urine or when supplying methane gas as an electron donor, it becomes possible to produce methanol from the methane gas, and to supply the produced methanol to the denitrifying bacteria 13 so as to bring the activation more efficiently. Therefore, it is possible to convert effectively the nitrate ions 14 and the nitrite ions 14' into nitrogen gas 15, so as to make them harmless.

By supplying methane gas into complex microorganisms (activated sludge), it is possible to allow microorganisms capable of utilizing methane gas among the complex microorganisms to create an organic matters, and then to allow the denitrifying bacteria to make the denitrogenation using the organic matters. Therefore, without converting methane gas into methanol and then giving the methanol as an electron donor to the denitrifying bacteria, it is possible to cause the denitrogenation reaction by activating the denitrifying bacteria.

The positional relationship between the oxygen supplying apparatus 20 and the electron donor supplying apparatus 1 in soil is desirable to satisfy that the oxygen supplying apparatus 20 is located as near as the microbial activity controlling material supplying apparatus 1 unless the oxygen is supplied to anaerobic microorganism residing ambient region around the microbial activity controlling material supplying apparatus 1, and so that the nitrite ions produced by the ammonia-oxidizing bacteria 23, and the nitrate ions produced by the nitrite-oxidizing bacteria can be removed by the denitrifying bacteria 13. For instance, by digging a hole or ditch of about several centimeters in depth which reaches the position for embedding, laying the bags 4 in the hole, and pouring soil over the bags in order to embed the bags 4 in soil, and using the bags in soil, it is possible to remove ammonia, nitrite ions, nitrate ions from the soil where the bag 4 is embedded or from the ground water flowing or permeating through the soil, and to convert them into harmless nitrogen gas. As useable location , places where a large volume of ammonia, nitrite ions, or nitrate ions are included in ground water, for example, farm lands where chemical fertilizers are frequently used, or disposal sites for farm animals' waste and urine, are enumerated. Separately, by embedding into sludge, it is possible to activate the microorganisms in the sludge so as to accelerate the removing of pollutant. When removing ammonia, nitrite ions, or nitrate ions from the soil or ground water by such a method, it become possible to realize the supply of water for living use at the place where most of the water for living use is depended on ground water. Further, it is particularly useful as the countermeasures against the nitrogen load to the nature which is the worldwide serious problem.

Separately, in the case that anaerobic microorganisms can not be activated since the ambient region around the embedded electron donor supplying apparatus 1 is aerobic circumstance, by burying a microbial activity controlling material supplying apparatus in which an oxygen absorbing material 21 is filled (hereinafter, the apparatus is referred as "oxygen absorbing apparatus 25") as shown in Fig. 9, the ambient region around the microbial activity controlling material supplying apparatus 1 can be changed to anaerobic condition, and the anaerobic microorganisms can be activated. As the oxygen absorbing material 21, any material which can absorb oxide and does not show a corrosive action to the non-porous film 2 can be used, for instance, solid reducing agents such as reduced iron, and solution including the reducing agent such as sodium sulfite solution are enumerated, however, the oxygen absorbing agent is not limited to the above material. As mentioned above, when burying the oxygen absorbing material filling apparatus 25 which has as a part the non-porous film 2 and in which oxygen absorbing material is filled into soil, it becomes possible to make, a place which tends to become the aerobic circumstance, such as a place near the Earth's surface, and a place into which ground water containing rich oxygen flows or permeates constantly, anaerobic condition for a longtime, and thus it is possible to activate the anaerobic microorganisms under a preferable environment.

Further, the microbial activity controlling material supplying apparatus according to the present invention can be used for dechlorinating organic chlorinated compound and making it harmless.

For instance, when aerobic organic microorganisms such as phenol-degrading bacteria, toluene-degrading bacteria and methane-degrading bacteria, etc., are activated with the oxygen supplying apparatus 20, while enzyme is induced by supplying an electron donor material at a minimum amount for sustaining lives of the phenol-degrading bacteria, the toluene-degrading bacteria and the methane-degrading bacteria with the aid of controlling film material, film thickness and film density of non-porous film 2 in the electron donor supplying apparatus 1, trichloroethylene can be dechlorinated by co-metabolism of oxidizing enzymes which are produced by the above bacteria, so as to produce dichloroethylene and vinyl chloride, and finally, they can be converted to ethylene, and thus it is possible to make the organochlorine compound harmless.

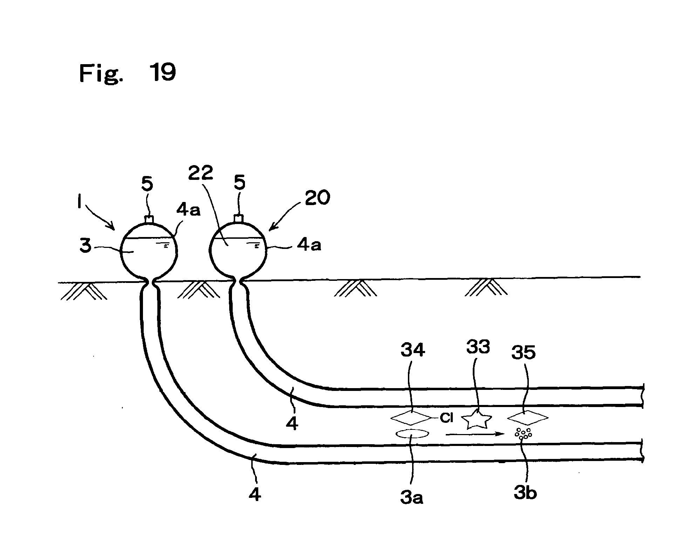

Now, it will be more concretely described. Although the fashion of embedding the oxygen supplying apparatus 20 and electron donor supplying apparatus in not particularly limited, for example, as shown in Fig. 19, the oxygen supplying apparatus is set close to the electron donor supplying apparatus 1 to the extent of providing an aerobic circumstance around the electron donor supplying apparatus 1. By embedding them in such condition, it becomes possible to maintain the region around the electron donor supplying apparatus 1 as aerobic circumstance for a long period. Then, under this aerobic circumstance, by supplying the electron donor material 3' in minute doses and at a gentle rate to the microorganisms residing at ambient region around the vessel 4 of the electron donor supplying apparatus 1, so as to stimulate the microorganisms, the lives of microorganisms can be maintained. On this occasion, in some aerobic microorganisms (phenol-degrading bacteria, methane- degrading bacteria, toluene-degrading bacteria, ammonia-oxidizing bacteria, etc.), metabolic enzyme 33 is induced due to the above stimulation. Therefore, by co-metabolism, the electron donor material 3a can be degraded so as to produce a degradation product 3b, while the organic type chloride compounds 34, such as trichloroethylene, dichloroethylene, vinyl chloride, etc., which are environment polluting compounds, can dechlorinated so as to produce dechlorinated compounds, and finally, these compounds can be converted to ethylene. Thus, it is possible to make the compounds harmless.

The co-metabolism is the phenomenon that in the case that the metabolizing enzyme induced by an interested microorganism possesses a broad substrate specificity, the microorganism also degrades a certain substance which is not the inherent decomposition target material for the microorganism. For instance, with respect to the methane- degrading bacteria, methane monooxygenase which is produced by the methane-degrading bacteria is a enzyme for decomposing methane inherently. However, this enzyme possesses a broad substrate specificity, and thus it can accerelate the dechlorination of trichloroethylene. If the methane as an electron donor material is not supplied, however, the metabolizing enzyme is not induced, and the methane-degrading bacteria will die after a while. If the supply of methane as electron donor material is too large, the dechlorination reaction for trichloroethylene becomes difficult to be caused. Namely, the decomposition reaction of methane which is the inherent energy source material for the methane-degrading bacteria and the decomposition reaction of materials, such as trichloroethylene, which are not an electron donor material for the methane-degrading bacteria are competitive to each other. Thus, when supplying the electron donor material at a minimum dose for sustaining lives of microorganisms such as methane-degrading bacteria, phenol-degrading bacteria, and ammonia-oxidizing bacteria, which can degrade organic chrolinated compounds by the co-metabolism, so as to stimulate the microorganisms and induce the enzyme metabolism, it is possible to perform decomposition treatment of organic chlorinated compound effectively.

In the case that only the oxygen supplying apparatus 20 is embedded into soil, it is also possible to remove phenol, toluene and benzene by activating aerobic microorganisms in soil such as phenol-degrading bacteria, toluene-degrading bacteria, and benzene-degrading bacteria. Further, by activating aerobic microorganisms in the soil which is polluted by petroleum type hydrocarbons such as heavy oil and gasoline, it is possible to remove the pollution. Further, by activating aerobic microorganisms such as phenol-degrading bacteria, toluene-degrading bacteria, and methane-degrading bacteria, which utilize as electron donor materials the materials degraded from phenol, toluene, and methane existed in the soil, trichloroethylene can be dechlorinated by co-metabolism of oxidizing enzymes which are produced by the above bacteria, so as to produce dichloroethylene and vinyl chloride, and finally, they can be converted to ethylene, and thus it is possible to make the trichloroethylene harmless. Separately, it is possible to remove VOC released from industrial waste buried in soil, by activating aerobic microorganisms in soil, such as phenol-degrading bacteria, toluene-degrading bacteria, and benzene-degrading bacteria.

*Dehalococcoides sp.* and *Methanosarcina sp.* are anaerobic microorganisms which can convert poly(tetra)chloro ethylene to tetrachloro ethylene, dichloro ethylene and vinyl chloride by dehalogenating respiration. In order to cause the dehalogenating respiration, electron donor materials are necessitated. Therefore, when embedding the electron donor supplying apparatus 1 under anaerobic condition and thus activating *Dehalococcoides sp.* and *Methanosarcina sp.,* it becomes possible to dechlorinate poly(tetra)chloro ethylene. Further, when embedding the electron donor supplying apparatus 1 along with an oxygen absorbing apparatus 25 and thus making the ambient region around the electron donor supplying apparatus 1 to aerobic circumstance, it becomes possible to dechlorinate poly(tetra)chloro ethylene.

Incidentally, when the electron donor material is provided at the minimum amount necessitated for maintaining the lives of microorganisms by regulating with film material, film thickness or film density of the non-porous film 2 of the electron donor supplying apparatus 1, it is possible to use the electron donor supplying apparatus so as to be on standby and not to activate the microorganisms which are resisting at the ambient region around the electron donor supplying apparatus 1 and require the electron donor material, while preventing the microorganisms from dies.

Further, the microbial activity controlling material supplying apparatus according to the present invention can be used for improving and modifying the soil where the desertification has been advanced by soil exhaustion due to over - grazing, over - cutting, over - land - reclaiming. According to the present invention, since the material necessitated for the microorganisms' activities can be supplied with a gentle rate, it is possible to activate microorganisms in the soil and then to improve and modify the soil by various polymers released by the activated microorganisms.

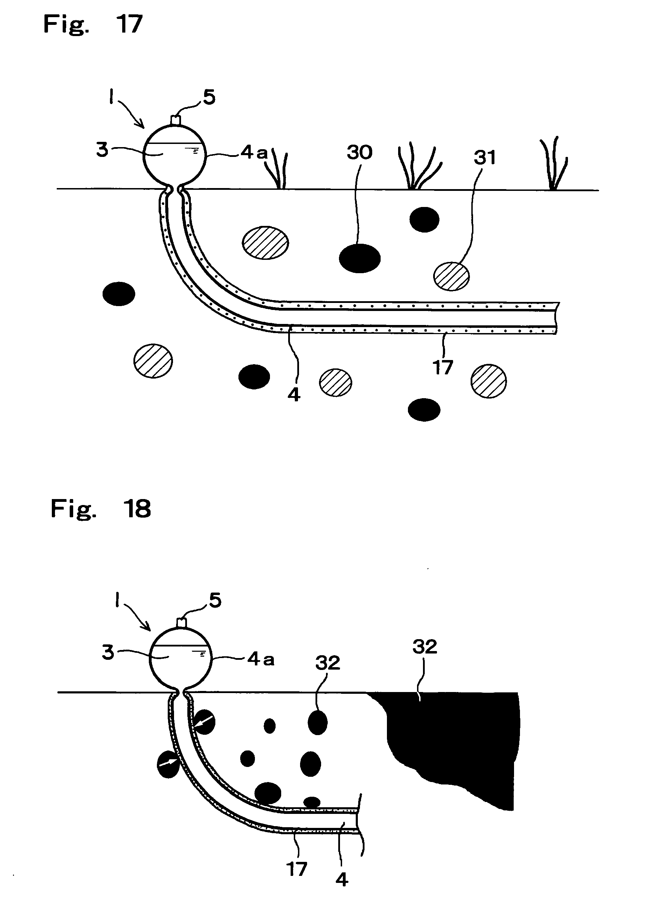

More concretely, as the microbial activity controlling material, inorganic salts of nitrogen and phosphorus, for instance, nitrates such as potassium nitrate, phosphates such as potassium phosphate, ammonium salts such as ammonium sulfate, are packed in the vessel 4 along with the electron donor material. As the non-porous film which constitutes the vessel 4, a hydrophilic film or an amphipatic film is used. By embedding thus constructed microbial activity controlling material supplying apparatus 1 into the soil where the desertification has been advanced, as shown in Fig. 17, it is possible to activate the microorganisms in the soil by giving them the electron donor materials, as well as to enhance the concentrations of the inorganic salts in the soil where the desertification has been advanced and the inorganic salts are lacking. Then, by activating the soil microorganisms, bio-polymers are produced, and the bio-polymers are accumulated in the soil. Therefore, the aggregate of the soil which is fitted for plants to grow can be formed, and the inorganic salts of such as metals, nitrogen and phosphorus, and water can be retained easily to the soil. Thus, the improvement of the soil can be attained. As the polymer produced by the soil microorganisms, concretely, for example, ε-polylysine (ε-PL) which is a basic amino acid polymer functions as coagulating agent 30 may be mentioned. This polymer is produced by the soil microorganisms such as *Rodococcus sp.* Further, Y-polyglutamic acid (PGA) which is a water absorbent polymer 31 is also produced by the soil microorganisms. This polymer is produced by the soil microorganisms such as Bacillus sp. Furthermore, cellulose composed by polysaccharide, etc., also produced by the soil microorganisms.

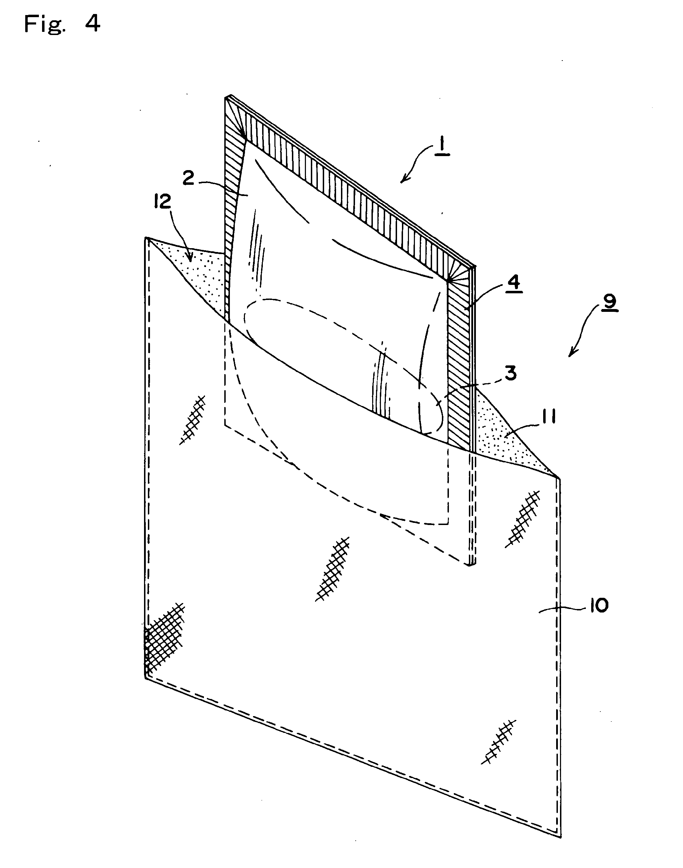

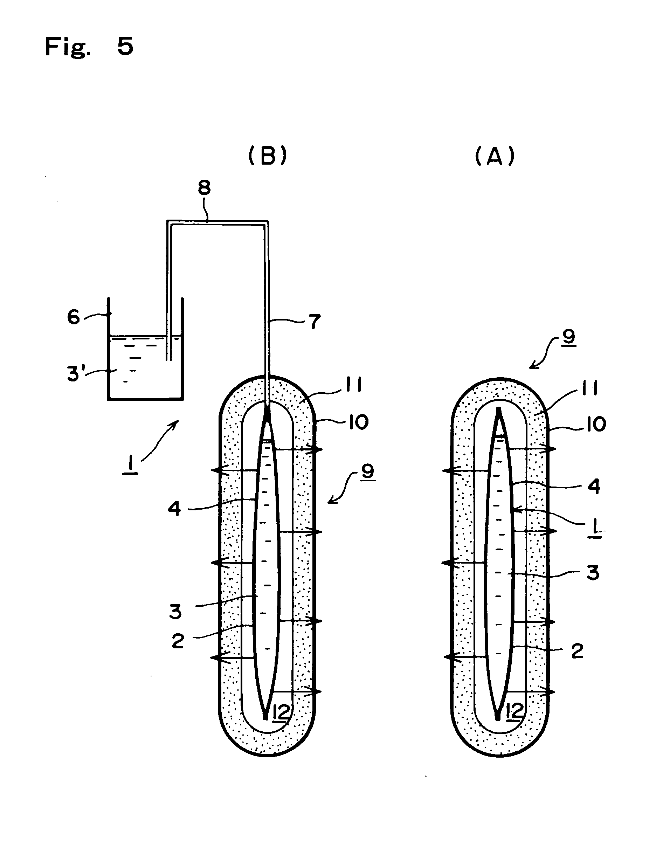

The microbial activity controlling material supplying apparatus 1 according to the present invention can be utilize all of bioreactors in which the microbial activity controlling material is supplied in order to control the activities of microorganisms. In the embodiment shown as follows, although the case that as the microbial activity controlling material the electron donor material is used and as the microorganisms the ammonia-oxidizing bacteria and the denitrifying bacteria are used will be described, the microbial activity controlling material supplying apparatus according to the present invention is not limited to such embodiment. Namely, when using microorganisms which can remove the intended components, other than the above mentioned microorganisms, it becomes possible to remove the components other than the nitrogen compounds such as ammonia, nitrate ions and nitrite ions. The bioreactor 9 of the embodiment shown in Fig. 4 and Fig. 5(A) comprises a carrier 11 which bears the ammonia-oxidizing bacteria and the denitrifying bacteria; a bag 10 which is made of a non-woven fabric and which intends to give a structural support of the carrier; and a vessel 4 as the electron donor supplying apparatus which is stored in a pocket formed at the inner side of the bag-shaped carrier 11 which is supported by the bag 10 of the non-woven fabric, and which is filled with the electron donor material 3 closely so as to supply autonomously and gently at a constant rate the material which functions as an electron donor for the energy source of the denitrifying bacteria immobilized on the carrier 11 to the denitrifying bacteria. The carrier 11 is coated on the inner side surface or outer side surface of the bag 10 of the non-woven fabric, so as to sustain the bag 10 of the non-woven fabric as a supporting structure. As the carrier, it is possible to use the same material with that for a carrier 17 which is provided on the surface of the microbial activity controlling material supplying apparatus 1. Incidentally, non-woven fabric, nylon net, etc., may be utilized for reinforcing the carrier, but the material is not limited thereto.

In the case of the bioreactor which will be used in gas phase, it is preferable to use a water absorbent polymer as the carrier 11. In such case, since the water holding ability can be further enhanced, when removing a intended component from the atmosphere by setting the bioreactor in the atmosphere, the intended component can be removed by allowing the intended component to be solved in the water included in the gel. For example, in the case of removing ammonia gas, when the ammonia gas is attached to the surface of the bioreactor, it becomes possible to enhance the ammonia gas removing efficiency because the attached ammonia gas tends to convert to ammonia ions easily. Further, when the immobilized microorganisms are dried, there is a fear that they will die out. Thus, when using in the atmosphere, it is necessary to supply water at predetermined intervals. However, when the water absorbent polymer is used, the labor for the water supplying can reduce extremely, or can exclude completely if the water supplying can be accomplished only by the water content in the atmosphere. Further, when the bioreactor is used in soil or in ground water, it is also possible to absorb and hold the water efficiently, and thus possible to remove the environment polluting material for a long period. As the water absorbent polymer, it is possible to use the same material with that for the carrier 17 provided on the surface of the microbial activity controlling material supplying apparatus 1.

Incidentally, the carrier 11 is not limited to the above mentioned ones, and it may be also possible, for example, to apply a napping treatment to the non-woven fabric so that the microorganisms can be attached directly to the surface, or to apply a napping treatment to the surface of the non-porous film 2 in order to bear the microorganisms thereon.

As the ammonia-oxidizing bacteria and the denitrifying bacteria, any species known in this art can be utilized. More concretely, for example, the followings can be enumerated as the ammonia-oxidizing bacteria:
*Nitrosomonas europaea* IFO-14298,
*Nitrosomonas europaea, N. marina*,*
*Nitrosococcus oceanus*, N. mobilis,*
*Nitrosospira briensis,*
*Nitroso lobus multiformis,* and
*Nitrosovibrio tenuis*; and
as the denitrifying bacteria:
*Paracoccus dinitrificans* JCM-6892**,
*Paracoccus denitrificans**,*
*Alcaligenes eutrophus**, A. faecalis,*
*Alcaligenes sp.* Ab-A-1, Ab-A-2, G-A-2-1 (FERM P-13862, P-13860, P-13861)*
*Pseudomonas denitrificans,*
*Thiosphaera pantotropha***,*
*Thiobacillus denitrificans***.*

Further, It is possible to bear nitrite-oxidizing bacteria in addition to the ammonia-oxidizing bacteria and the denitrifying bacteria. As the nitrite oxidizing bacteria, any species known in this art can be utilized. More concretely, for example, the followings can be enumerated:
*Nitrobacter winogradskyi, N. hamburgensis,*
*Nitrospina gracilis*,*
*Nitrococcus mobilis*,* and
*Nitrospira marina*.*

Incidentally, the strains marked with an asterisk * in above enumeration are the strains which can be used only for treatment of sea water. In *N. europaea* and *N. winogradskyi,* the ones which can be used in plain water and the ones which can be used in sea water exist. The strains to which the deposited number is added are the strains which has been already deposited by this applicant. The strains marked with two asterisk ** in above enumeration are the strains which can use hydrogen as the energy source. The strains marked with three asterisk *** in above enumeration are the strains which can use only sulfur as the energy source, and which are capable of performing the denitrification using a sulfur compound such as hydrogen sulfide, etc.

The ammonia-oxidizing bacteria are aerobic microorganisms which convert (oxidize) ammonia (ammonium ions) to nitrite ions, and the denitrifying bacteria are anaerobic microorganisms which convert (reduce) nitrate ions or nitrite ions to nitrogen gas. Namely, when using the bioreactor which bears the ammonia-oxidizing bacteria is used under an aerobic condition, it become possible to convert ammonia to nitrite ions, and possible to repress odor. Separately, when using bioreactor which bears the denitrifying bacteria under an anaerobic condition, it is possible to convert nitrate ions or nitrite ions to harmless nitrogen gas. When using these microorganisms in combination, it becomes possible to remove ammonia, nitrate ions and nitrite ions. Incidentally, when supplying oxygen to the ammonia-oxidizing bacteria, although the function of the denitrifying bacteria which are anaerobic microorganisms would be repressed, the denitrification of the denitrifying bacteria may be performed by maldistributing the denitrifying bacteria locally at a preferable region in the bioreactor, i.e., an anaerobic region apart from the oxygen supplying position. The nitrite-oxidizing bacteria are the microorganisms which oxidize the nitrite ions to nitrate ions, and thus when it is carried on the bioreactor the denitrification reaction can be performed more effectively.

The above enumerated strains may be immobilized on the carrier singly, or in combination of two or more of isologous or heterologous strains. Further, it may immobilize the microorganism resisting in sludge per se. For instance, microorganisms such as *Achromobacter* and *Alcaligenes* in activated sludge, or phosphorous removing microorganisms and iron bacteria in waste water, per se, and also microorganisms which can aid the growth of the above mentioned microorganisms, can be used.

According to the bioreactor constituted as above, it is possible to supply the electron donor material 3 uniformly to the surface on which microorganisms which requires the electron donor, i.e., the denitrifying bacteria are immobilized, by allowing the electron donor material 3 filled in the vessel 4 to permeate through the non-porous film 2 and to release outwardly to the ambient region around the vessel 4, namely, to release outwardly to the bag (pocket 12) of carrier 11 surrounding the vessel 4, and to diffuse in the bag of the carrier 11. Namely, when filling the electron donor material 3 near the microorganisms, it becomes possible to supply the electron donor material autonomously, uniformly, and with a gentle sustained-release. Since the bioreactor is composed by merely inserting the electron donor supplying apparatus which is filled with the electron donor material 3 into the bag 10 of the carrier, on the occasion of consuming the electron donor material 3 sealed in the bag, by replacing to a fresh bag which is sealed and is filled with the electron donor material, it is possible to remove ammonia, nitrate ions and nitrite ions in the region to be treated, continuously, with converting them to nitrogen gas. Incidentally, even if a liquid to be treated is filled around the vessel 4, the denitrifying bacteria can function as far as the electron donor material 3 is supplied by the permeation of the material 3 through the non-porous film 2. Further, when exposing the outer side surface of the bioreactor to air, it becomes possible to activate the ammonia-oxidizing bacteria and to remove the ammonia efficiently.

Further, as shown in Fig. 5(B), instead of the vessel 4 as the electron donor supplying apparatus in the bioreactor 9 of the embodiment shown in Fig. 4, it is also possible to use a electron donor supplying apparatus in which the closed bag-shaped vessel where a means for introducing the electron donor material 3 is provided as shown in Fig. 2, and the liquid electron donor material 3 can be refilled from outside as shown in Fig.3. In this case, as long as the liquid electron donor material, for instance, undiluted solution of alcohol 3' is reserved in the tank 6, the alcohol 3' can be refilled from the tank 6 by free fall as the volatile organic material 3 in the bag-shaped vessel 4 decreases. Thus, it is possible to perform the removal of the intended compound in the fluid to be treated, for instance the removal of ammonia for a long time, regardless the sizes of vessel or bag 4. Further, in this case, it is not necessitated to control and monitor mutually independently the numerous electron donor supplying apparatuses which are connected to the tank 6, but only to monitor the alcohol 3' in the tank 6 and to add it appropriately.

Further, the bioreactor can be constituted in the form of integrating the above mentioned vessel 4 of the electron donor supplying apparatus and the carrier 11 on which the microorganisms effective for removing the intended component are immobilized. For instance, the non-porous film 2 part of the vessel 4 for sealing the electron donor material 3 is stuck to the carrier 11 in order to constitute the bioreactor in which the carrier 11 and the electron donor supplying apparatus are integrated. In such case, since the microorganisms are immobilized on the face through which the electron donor material 3 permeates, the whole amount of the electron donor material supplied is supplied directly to the microorganisms.

The bioreactor according to the present invention can be placed to a decontamination targeting area to remove the environmental pollutant. Further, in the case of the bioreactor to which aerobic microorganisms are carried, it is necessary to perform an aeration treatment for activating the aerobic microorganisms. In this case, the aeration treatment may be performed by placing the above mentioned oxygen supplying apparatus 20 near the bioreactor, and then supplying oxygen to the ammonia-oxidizing bacteria.

In addition, it is possible to provide inorganic ions to the carrier 11 of the bioreactor, by using a microbial activity controlling material supplying apparatus 1 to which inorganic salts as the microbial activity controlling material is filled, so as to form an environment for promoting the multiplication of microorganisms in the carrier. For instance, when the microbial activity controlling material supplying apparatus 1 to which both of the inorganic salts and the electron donor materials are filled as the microbial activity controlling materials is used, the multiplication rate of the microorganisms can be enhanced, while the environmental pollutant can be treated. Thus, the efficiency of removing the environmental pollutant may be further improved.

In addition, it is possible to control pH of the carrier 11 of bioreactor, by using a microbial activity controlling material supplying apparatus 1 to which acidic material(s) or basic material(s) as the microbial activity controlling material is filled. For instance, in case that a rising or falling in pH value in the carrier is caused by the treatment with the microorganisms and which may bring the activities of the microorganisms to a lessened level, by using a microbial activity controlling material supplying apparatus 1 to which both of the acidic material(s) or basic material(s), and the electron donor materials are filled as the microbial activity controlling materials is used, the environmental pollutant can be treated, while pH can be controlled to the value optimum for the microorganisms.

Further, it is possible to immobilize the microorganisms which can dechlorinate organic chlorine compounds to the carrier 11. As the dechlorinating microorganisms, any species known in this art can be utilized. More concretely, for example, the followings can be enumerated:
*Dehalococcoides sp.*
*Methanosarcina sp.*
These microorganisms can degrade tetrachloro ethylene, trichloroethylene, and dichloroethylene to vinyl chloride by the dechlorinating respiration, when they are activated by supplying hydrogen gas, methanol, or ethanol as the microbial activity controlling material under an anaerobic condition. That is, by immobilizing such microorganisms on the carrier 11, it is possible to obtain a bioreactor which can degrade the tetrachloro ethylene, trichloroethylene, and dichloro ethylene into vinyl chloride by the dechlorinating respiration under the anaerobic condition.

Furthermore, by utilizing co-metabolisms of the following phenol-degrading bacteria known in this art, the organic chlorine compound can be dechlorinated:
*Pseudomonas sp.,*
*Pseudomonas ptida,*
*Acinetobacter sp.,*
*Ralstonia sp.,*
*Ralstonia eutropha,*
*Azoarcus sp.,*
*Bacillus sp.,*
*Alcaligenes sp.,*
*Alcaligenes faecalis,* and
*Rhodococcus sp.*

Now, the co-metabolism by the phenol-degrading bacteria will be explained. The decomposition of phenol and the decomposition of organic chlorine compound(s) are competitive to each other. Thus, when the amount of phenol which is the inherent decomposition target material for the phenol-degrading bacteria is large, the organic chlorine compound is not degraded well. On the other hand, when the phenol are not supplied appropriately to the phenol-degrading bacteria, the metabolizing enzyme are not induced, and the phenol-degrading bacteria will die after a while. Thus, when supplying the phenol as the electron donor material in minute doses and gently, from the microbial activity controlling material supplying apparatus 1 to which the phenol is filled, it becomes possible to stimulate the phenol-degrading bacteria carried on the carrier 11 gently, so as to sustain the lives of the phenol-degrading bacteria and to induce the enzyme metabolism, and to degrade the organic chlorine compound effectively for a long time.

The enzyme produced by the phenol-degrading bacteria possesses broad substrate specificity, thus it can degrade trichloroethylene, dichloroethylene and vinyl chloride by dechlorinating them to ethylene, besides it degrades the phenol which is the inherent decomposition target material for this bacterial strain. Further, it is possible to degrade volatile organic material such as toluene, benzene, etc., so as to utilize the decomposition product as energy source. These strains may be immobilized singly or in combination of two or more strains. Further, it is also possible to combine these phenol-degrading bacteria with the methane-degrading bacteria, the toluene-degrading bacteria, or the ammonia-oxidizing bacteria which are known as microorganisms capable of dechlorinating organic chlorine compounds by co-metabolism. Namely, when immobilizing such microorganisms on the carrier 11, it becomes possible to provide a bioreactor capable of dechlorinating and trichloroethylene, dichloroethylene, and vinyl chloride so as to degrade them into ethylene which is harmless.

As the electron donor material to be supplied to the bioreactor which practices the dechlorination, any materials which can permeate the non-porous film 2 and can stimulate the microorganisms to keep them alive may be used. The followings are examples of microorganisms and electron donor materials corresponding to the respective microorganisms:
(a) Methane-degrading bacteria: methane
(b) Ammonia-oxidizing bacteria: ammonia
(c) Toluene-degrading bacteria: toluene
(d) Phenol-degrading bacteria: phenol, toluene, benzene.
   Thus, in accordance with the kind of microorganism immobilized on the bioreactor, the energy source material for stimulating the microorganism can be selected and used appropriately.

Incidentally, although the above mentioned embodiments are preferable embodiments of the present invention, but the present invention is not limited to these embodiments, and various changes and modifications can be adaptable without departing from the burden of the present invention. For instance, when the quantity of the ammonia-oxidizing bacteria residing in soil is not ample, it is possible to use a bioreactor in which ammonia-oxidizing bacteria is carried on the outer surface of the oxygen supplying apparatus 20 by burying it near the electron donor supplying apparatus, in order to remove ammonia, nitrate ions, and nitrite ions efficiently.

Sulfur oxides (SOₓ) in the atmosphere change to sulfur ions when the sulfur oxides are dissolved in water. Therefore, when providing a carrier 17 capable of bearing microorganisms onto the surface of the microbial activity controlling material supplying apparatus 1 and bearing *Desulfovibrio sp.,* etc., on the carrier 17, it becomes possible to remove the sulfur oxides in the atmosphere while supplying the electron donor material. Further, nitrogen oxides (NOₓ) in the atmosphere change nitrate ions when the nitrogen oxides are dissolved in water. Thus, when bearing the denitrifying bacteria on the carrier 17, it also becomes possible to remove the nitrogen oxides in the atmosphere.

Further, when providing a carrier 17 onto the surface of the microbial activity controlling material supplying apparatus 1 and bearing microorganisms, such as *Acinethactor sp.,* etc., which degrade waste oils and utilize the decomposition product as energy source, on the carrier 17, it becomes possible to practice the decomposition treatment of the spillage waste oil in ocean. In the case of performing the decomposition of the spillage waste oil by microorganisms, the addition of inorganic salts such as nitrogen containing salts and phosphorus containing salts is necessitated. However, even if such inorganic salts are added to ocean, the inorganic salts are dispersed very soon, and thus, it is difficult to supply the inorganic salts to the microorganism. On the other hand, when solutions of nitrates, ammonium salts, and/or phosphates as the microbial activity controlling material are stored closely in the microbial activity controlling material supplying apparatus and nitrogen and phosphorus of them are supplied to the microorganisms, it becomes possible to form an environment preferable for the activities and multiplications of microorganisms, and thus to perform the decomposition of waste oil effectively. Further, when the microbial activity controlling material supplying apparatus 1 is formed as a sheet of a large area as shown in Fig. 18, it can be also function as a sheet for preventing diffusion of the oil 32. Thus, it is possible to treat the spillage waste oil by microorganisms effectively, while preventing the diffusion of the spillage waste oil.

As mentioned above, by using the microbial activity controlling material supplying apparatus according to the present invention, it is possible to activate selectively only the microorganisms which own the function capable of removing the environment pollutants in the decontamination target region, among the microorganisms residing in the decontamination target region or the microorganisms added to the decontamination target region, and thus, possible to practice the bio-remediation with a low cost and briefly. Further, the microbial activity controlling material supplying apparatus according to the present invention can be applied in various scenes where the environmental pollutants are removed by using the microbial activity controlling material as energy source.

### EXAMPLES

### <Example 1>

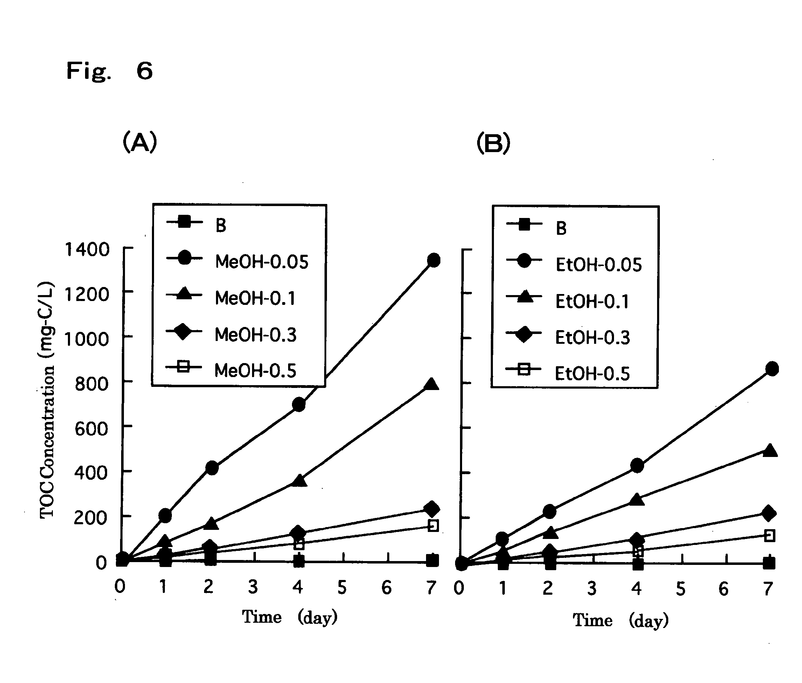

Bags were manufactured by using polyethylene film, and methanol or ethanol was added and sealed therein. Then, the bags were subjected to determination for the permeation volume of the organic matters in order to confirm the effectiveness of the microbial activity controlling material supplying apparatus according to the present invention.
Polyethylene films of 0.05 mm, 0.1 mm, 0.3 mm and 0.5 mm in thickness (Product Name: MIPOLON Film, manufactured by MITSUWA CO.,LTD) were shaped into bags, and methanol (manufactured by Wako Pure Chemical Industries, Ltd., 99.8 %) or ethanol (manufactured by Wako Pure Chemical Industries, Ltd., 99.5 %) was added to the bags and sealed therein. The liquid volume sealed in the individual bags was set to 5 mL. Then, the bags were dipped in water, and the permeation volumes of methanol and ethanol were evaluated by determining TOC concentration as the molecular permeation volume against the elapsed-date. The TOC concentration was measured by a combustion - infrared ray type total organic carbon analyzer (TOC-650, manufactured by TORAY Engineering Co. Ltd.). The obtained results are shown in (A) and (B) of Fig. 6. In the figures, B, MeOH, and EtOH represent the background, methanol, ethanol, respectively, and numerals such as 0.05, 0.1, 0.3 and 0.5 represent thickness (Unit: mm) of polyethylene film. From this experiment, it was confirmed that the supplying rate of the electron donor material can be controlled by the thickness of polyethylene film. Because, the thinner the thickness of the polyethylene film was, the more the TOC concentration increased.

### <Example 2>

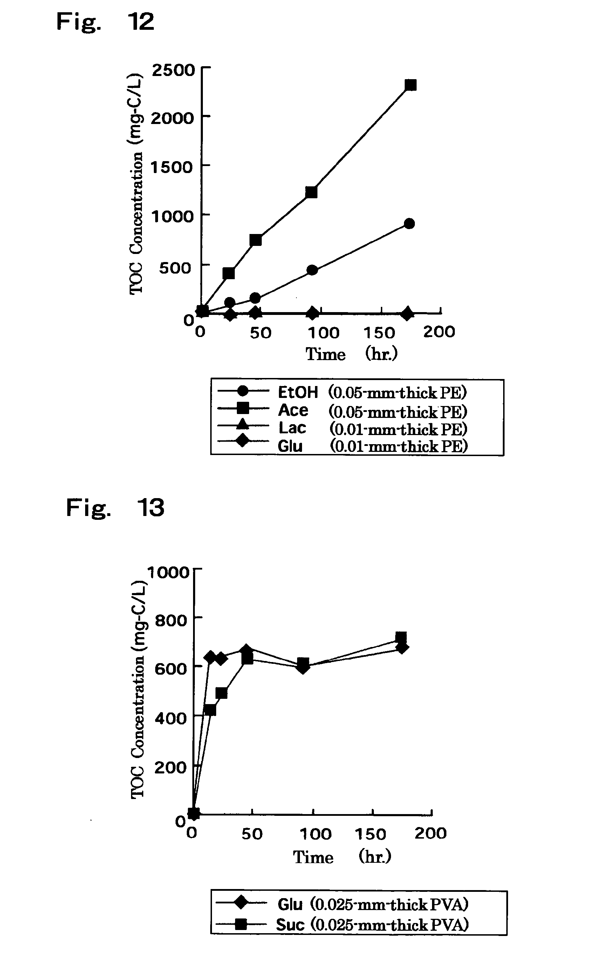

The permeability of acetic acid, lactic acid and glucose to polyethylene films were investigated. Polyethylene film of 0.05 mm in thickness (Product Name: MIPOLON Film, manufactured by MITSUWA CO.,LTD) was shaped into bags, and acetic acid (manufactured by Wako Pure Chemical Industries, Ltd., 99.7 %) was added to the bags and sealed therein. Separately, polyethylene film of 0.01 mm in thickness (Product Name: Polyethylene Wrap, manufactured by The Daiei, Inc.) was shaped into bags, and lactic acid (manufactured by Wako Pure Chemical Industries, Ltd., 85 - 92 % solution of DL-lactic acid) or glucose (manufactured by Wako Pure Chemical Industries, Ltd., 10 % aqueous solution) was added to the bags and sealed therein. The liquid volume sealed in the individual bags was set to 5 ml. Then, the bags were dipped in water, and the permeation volumes of acetic acid, lactic acid and glucose were evaluated by determining TOC concentration against the elapsed-time. In addition, with respect to a bag which was manufactured by the polyethylene film and to which ethanol (manufactured by Wako Pure Chemical Industries, Ltd., 99.5 %) was added and sealed therein, the similar measurement of the TOC concentration was performed in order to perform a comparison with the permeability of acetic acid, lactic acid and glucose. The obtained results are shown in Fig. 12. In the figure, EtOH, Ace, Lac, and Glu represent ethanol, acetic acid, lactic acid and glucose, respectively. From this experiment, it was confirmed that the permeation rate of acetic acid through the polyethylene film was faster than that of ethanol. On the other hand, it was confirmed that lactic acid and glucose hardly permeated the polyethylene film. Thus, it became evident that the acetic acid can permeate the polyethylene film in addition to alcohols such as methanol and ethanol, while it became evident that the materials easy - dissolvable to water such as lactic acid and glucose hardly permeate the polyethylene film.

### <Example 3>

The permeability of glucose and sucrose, which is a disaccharide of which molecular weight is larger than that of glucose which is a monosaccharide, to polyvinyl alcohol (PVA) film were investigated. Polyvinyl alcohol film of 0.025 mm in thickness (Product Name: Vinylon® Film DX-N #25, manufactured by TOHCELLO CO.,LTD) was shaped into bags, and glucose (manufactured by Wako Pure Chemical Industries, Ltd., 10 % aqueous solution) or sucrose (manufactured by Wako Pure Chemical Industries, Ltd., 10 % aqueous solution) was added to the bags and sealed therein. The liquid volume sealed in the individual bags was set to 5 mL. Then, the bags were dipped in water, and the permeation volumes of glucose and sucrose were evaluated by determining TOC concentration against the elapsed-date. The obtained results are shown in Fig. 13. In the figure, Glu and Suc represent glucose and sucrose, respectively. From this experiment, it was confirmed that glucose and sucrose permeated the PVA film. It was also confirmed that, with respect to the time elapsed for bringing the TOC concentration to the equilibrium condition, the time for the glucose was about 15 hours after starting, while the time for the sucrose was about 50 hours after starting, and thus, the time for sucrose was slower than the time for glucose. Therefore, when using a hydrophilic film such as PVA, it becomes possible to allow saccharides, such as glucose and sucrose, which have large molecular weights, to permeate the film. Further, since the sucrose required a more elongated time for bringing the TOC concentration to the equilibrium condition than the time required by glucose, wherein the sucrose is disaccharide of which molecular weight is larger than that of glucose which is a monosaccharide, it became evident that the molecular permeation rate through PVA film can be controlled by the molecular weight. Incidentally, in this experiment, a tendency that the permeation rates of glucose and sucrose through the PVA film were faster than the permeation rates of ethanol, methanol and acetic acid through the polyethylene film was observed. It is due to the fact that thickness of the used PVA film was a thin level of 0.025 mm. Thus, the permeation rates of glucose and sucrose can be repressed by thickening the thickness of the PVA film, or by using ethylene - vinyl alcohol copolymer film, which shows intermediate characteristics between those of polyethylene and those of PVA film.

### <Example 4>

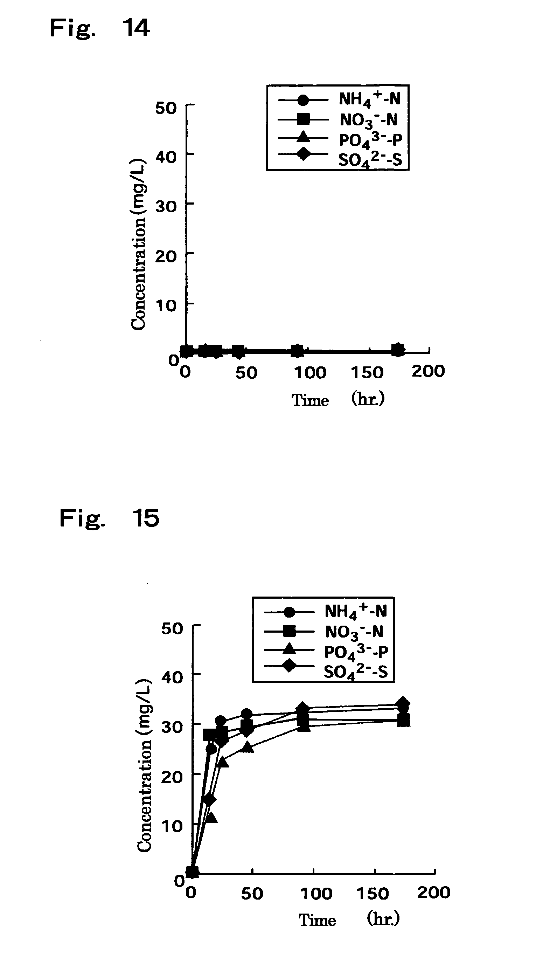

The permeability of ammonium ion (NH₄⁺), nitrate ion (NO₃-), phosphate ion (PO₄³⁻) and sulfate ion (SO₄²⁻) through polyethylene film were investigated.
Polyethylene film of 0.01 mm in thickness (Product Name: Polyethylene Wrap, manufactured by The Daiei, Inc.) was shaped into bags, and a solution containing ammonium ions and sulfate ions, a solution containing nitrate ions, or a solution containing phosphate ions was added to the bags and sealed therein. The solution containing ammonium ions and sulfate ions had been prepared by dissolving ammonium sulfate 0.047 g to distilled water 10 mL so as to become a concentration of 1000 mg-N/L. The solution containing nitrate ions had been prepared by dissolving potassium nitrate 0.072g to distilled water 10 mL so as to become a concentration of 1000 mg-N/L. The solution containing phosphate ions had been prepared by dissolving potassium phosphate 0.056g to distilled water 10 mL so as to become a concentration of 1000 mg-P/L. Then, the bags in which the solutions were sealed were dipped in water, and the permeation rates of ammonium ion, nitrate ion, phosphate ion and sulfate ion were evaluated by determining ion concentration against the elapsed-time. The ammonium ion concentration was measured in accordance with the indophenol blue absorptiometry. The concentrations of other ions were measured by an ion chromatogram analyzer (DX-AQ, manufactured by Dionex Corporation). The obtained results are shown in Fig. 14. In this figure, NH_{4⁺}-N, NO_{3⁻}-N, PO_{4³⁻}-P and SO_{4²⁻}-S represents, ammonium ion, nitrate ion, phosphate ion and sulfate ion, respectively. From this experiment, it was confirmed that ammonium ion, nitrate ion, phosphate ion and sulfate ion hardly permeate the polyethylene film. Thus, it became evident that the permeation of ions is hardly realized by using the polyethylene film.

### <Example 5>

The permeability of ammonium ion (NH_{4⁺}), nitrate ion (NO_{3⁻}), phosphate ion (PO_{4³⁻}) and sulfate ion (SO_{4²⁻}) through polyvinyl alcohol (PVA) film were investigated.
PVA film of 0.025 mm in thickness (Product Name: Vinylon® Film DX-N #25, manufactured by TOHCELLO CO.,LTD) was shaped into bags, and a solution containing ammonium ions and sulfate ions, a solution containing nitrate ions, or a solution containing phosphate ions was added to the bags and sealed therein. These solutions were the same with those in Example 4. Then, the bags were dipped in water, and the permeation rates of ammonium ion, nitrate ion, phosphate ion and sulfate ion were evaluated by determining ion concentration against the elapsed-time in accordance with the same methods as in Example 4. The obtained results are shown in Fig. 15. From this experiment, it was confirmed that ammonium ion, nitrate ion, phosphate ion and sulfate ion permeate the PVA film. Further, it was also confirmed that, with respect to the time elapsed for bringing the respective ion concentration to the equilibrium condition, the times for the ammonium ion and for nitrate ion were about 50 hours after starting, while the times for the phosphate ion and for the sulfate ion were about 100 hours after starting, and thus, the times for the phosphate ion and for the sulfate ion were slower than the time the ammonium ion and for nitrate ion. Therefore, it became evident that the permeation of water-soluble ions such as ammonium ion, nitrate ion, phosphate ion, sulfate ion, etc., can be realized by using a hydrophilic film such as PVA film, etc. Further, since each of phosphate ion and sulfate ion required more elongated time for bringing the respective ion concentration to the equilibrium condition than the time required by each of ammonium ion and nitrate ion, wherein each of the phosphate ion and the sulfate ion has a larger molecular weight than those of the ammonium ion and nitrate ion, it became evident that the ion permeation rate through PVA film can be controlled by the molecular weight of ion. Incidentally, in this experiment, a tendency that the permeation rates of various ions through the PVA film were faster than the permeation rates of ethanol, methanol and acetic acid through the polyethylene film was observed. It is due to the fact that thickness of the used PVA film was a thin level of 0.025 mm. Thus, the permeation rates of various ions can be repressed by thickening the thickness of the PVA film, or by using ethylene - vinyl alcohol copolymer film, which shows intermediate characteristics between those of polyethylene and those of PVA film.

As mentioned above, from the results of permeation experiments for various materials using polyethylene films and polyvinyl alcohol films which are non-porous films, it became evident that the permeation rate through the non-porous film can be controlled by the characteristics of non-porous film and the characteristics of material molecule, the molecular weight of the material, and/or the thickness of the non-porous film.

Further, it also became evident that the permeation of methanol, ethanol and acetic acid, which are molecules each having a certain hydrophilic group, can take place even if a hydrophobic film such as polyethylene film is used. Thus, the molecules which have more carbon numbers and more hydrophobic than methanol molecule, ethanol molecule or acetic acid molecule tend to permeate the hydrophobic film more easily. Further, since molecules bearing no hydrophilic group, such as benzene, toluene, etc., shows a high affinity to the hydrophobic film, the hydrophobic film is easy to allow these molecules to permeate. In addition, when using the ethylene - vinyl alcohol copolymer (EVOH) film, which shows an intermediate characteristicsbetween those of polyethylene and those of PVA film, it becomes possible to allow the permeation of the microbial activity controlling material, regardless the hydrophilicity or the hydrophobicity for the molecule of the material.

### <Example 6>

The function of bioreactor was confirmed by using the polyethylene bag into which ethanol was sealed, as used in Example 1.

### (Strains to be tested and cultivation thereof)

*Nitrosomonas europaea* IFO-14298 as ammonia-oxidizing bacteria, and *Paracoccus denitrificans* JCM-6892 as a denitrifying bacterium were used. On the cultivation, a liquid medium of which basal composition is in accord with the IFO Medium List No. 240 was used for the *Nitrosomonas europaea,* and a liquid medium of which basal composition is in accord with the JCM Medium List No.22 (Nutrient agar No. 2) was used for the *Paracoccus denitrificans.* The compositions of these media are shown in Table 1. The former medium was prepared by adding Phenol Red as a pH indicator to the IFO medium No. 240, and adjusting pH with adding Na₂CO₃ appropriately instead of using CaCO₃. The latter medium was prepared as a liquid medium by omitting agar from the JCM medium No. 22. After shake (110 rpm) culture at 30 °C, the cultured bacteria were individually collected by centrifugation, and triple rinsed with a phosphate buffer solution (9 g/l Na₂HPO₄·12H₂O, 1.5 g/l KH₂PO₄, pH = 7.5). Then, the rinsed bacteria were suspended to the phosphate buffer solution so as to be a concentration of 8 mg (in dry wt.)/ml as for the *Nitrosomonas europaea,* and a concentration of 33 mg (in dry wt.)/ ml as for the *Paracoccus denitrificans.*

**[Table 1]**

| Medium for *N. europaea* (pH 8.0) | | Medium for *P. denitrificans* (pH 7.0 - 7.2) | |
|---|---|---|---|
| (NH₄)₂SO₄ | 0.5 g/l | Peptone | 10 g/l |
| NaCl | 0.3 g/l | Broth | 10 g/l |
| K₂HPO₄ | 1.0 g/l | NaCl | 5 g/l |
| M_{g}SO₄·7H₂O | 0.3 g/l | | |
| FeSO₄ ·7H₂O | 0.03 g/l | | |
| Phenol Red | 2 mg/l | | |

### (Bacteria immobilization method)

1 ml of the above mentioned *N europaea* suspended solution and 2 ml of the above mentioned *P. denitrificans* suspended solution were added to 9 ml of photo-cross-linkable resin PVA-SbQ (SPP-H-13, manufactured by Toyo Gosei Co., Ltd.) for immobilization. the mixed solution of the bacteria and the resin was coated directly on non-woven fabric, and then the coated non-woven fabric was exposed under irradiation of metal halogen lamp for 1 hour in order to form an immobilized carrier (length: 50 mm, width: 50 mm, thickness: 20 mm) in sheet form on the one surface of the non-woven fabric.

### (Performance evaluation)

Performance evaluation was done for a bioreactor equipped the immobilized carrier under the condition that the electron donor supplying apparatus in which ethanol was sealed into polyethylene film was used in the bioreactor.
The product prepared by above Example wherein a carrier embedding both *Nitrosomonas europaea* IFO-14298 and *Paracoccus denitrificans* JCM-6892 had been formed as a sheet onto the one surface of the non-woven fabric was set so that the non-woven fabric faced to the outside, and that a pocket for the microbial activity controlling material supplying apparatus was provided to the carrier. Further, a bag-shaped microbial activity controlling material supplying apparatus where ethanol was sealed in polyethylene film of 0.05 mm or 0.3 mm in thickness was inserted in the pocket, and then ammonia waste water treatment ability was evaluated.

### (Analyzing method)

The concentrations of ammonia and nitrite in the medium solution were determined in accordance with the indophenol blue absorptiometry, and the naphthyl amine absorptiometry, respectively.

### (Results of performance evaluation for bioreactor equipped the immobilized carrier under the condition that the electron donor supplying apparatus in which ethanol was sealed into polyethylene film was used in the bioreactor)

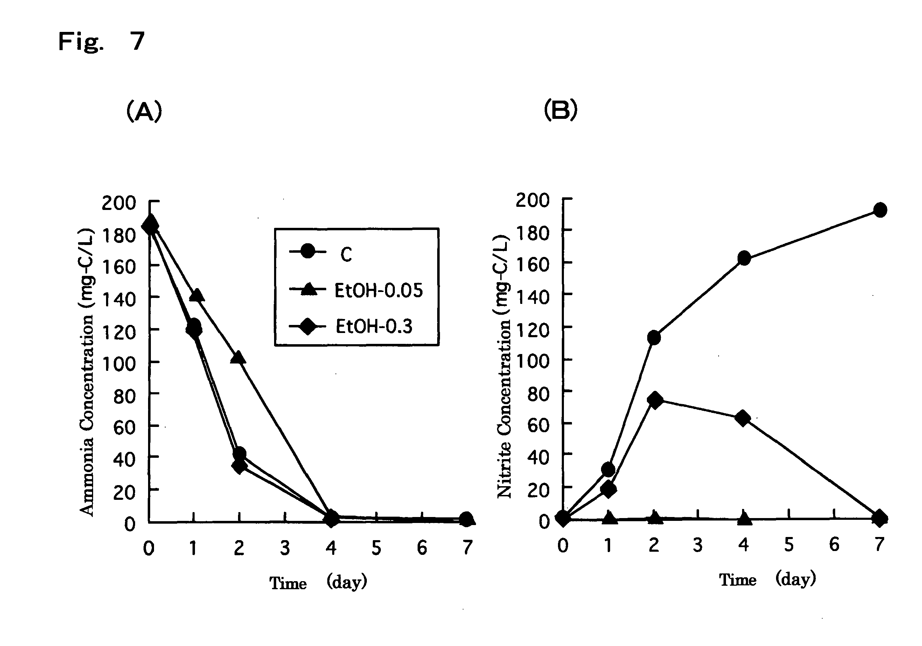

The result of performance evaluation for the bioreactors is shown in Fig. 7. In this figure, C denotes the result for the bioreactor without supplying the electron donor, EtOH-0.05 denotes the result for the bioreactor using a microbial activity controlling material supplying apparatus where ethanol was sealed in polyethylene film of 0.05 mm in thickness, and EtOH-0.3 denotes the result for the bioreactor using a microbial activity controlling material supplying apparatus where ethanol was sealed in polyethylene film of 0.3 mm in thickness. On the fourth day, the ammonia concentration reached zero for all cases of C, EtOH-0.05 and EtOH-0.3, while, the nitrite concentration, as for the case of C, had been rising until the seventh day. As for the case of EtOH-0.05, the nitrite concentration had been rising until the second day, thereafter decreasing, and it was not detected on the seventh day. As for the case of EtOH-0.3, the nitrite concentration was not detected from the first day. Thus, it was confirmed that the ethanol is supplied to the bioreactor through the polyethylene films, and also confirmed that the thinner the polyethylene films is, the easier the supplying of ethanol becomes.

### <Example 7>

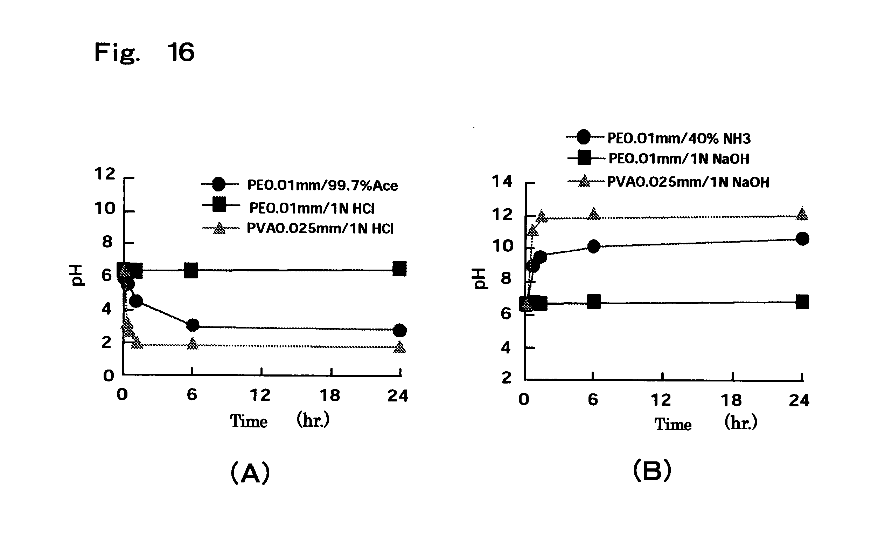

The pH controlling ability to the ambient region around polyethylene film or PVA film due to the permeation of an acidic material such as hydrochloric acid and acetic acid or a basic material such as sodium hydroxide and ammonia through the polyethylene film or PVA film was investigated.
Polyethylene film of 0.01 mm in thickness (Product Name: Polyethylene Wrap, manufactured by The Daiei, Inc.) was shaped into bags, and HCl solution (manufactured by Wako Pure Chemical Industries, Ltd., 1N), acetic acid (manufactured by Wako Pure Chemical Industries, Ltd., 99.7 %), NaOH solution (manufactured by Wako Pure Chemical Industries, Ltd., 1N), and ammonia solution (manufactured by Wako Pure Chemical Industries, Ltd., 25 %) were added individually to the bags and sealed therein. Separately, PVA film of 0.025 mm in thickness (Product Name: Vinylon® Film DX-N #25, manufactured by TOHCELLO CO.,LTD) was shaped into bags, and HCl solution (manufactured by Wako Pure Chemical Industries, Ltd., 1N) and NaOH solution (manufactured by Wako Pure Chemical Industries, Ltd., IN) were added individually to the bags and sealed therein. Then, the bags were dipped in water, and the permeability of acidic materials and basic materials through the polyethylene film or the PVA film were evaluated by determining pH against the elapsed-time. The obtained results are shown in Fig. 16. From this experiment, it was confirmed that the acetic acid which is an acidic material and the ammonia which is a basic material can permeate the polyethylene film and can control pH of the ambient region around the bag. Further, it was also confirmed that the hydrochloric acid which is an acidic material and the sodium hydroxide which is a basic material can permeate the PVA film and can control pH of the ambient region around the bag, although they hardly permeate the polyethylene film. Thus, it become evident that pH of the ambient environment around the bag can be changed to the desired pH and the desired pH can be maintained for a long period, by controlling the permeation of acidic material or basic material with the selection of the film among polyethylene film, PVA film and ethylene-vinyl alcohol copolymer film which shows an intermediate characteristics between those of polyethylene and those of PVA film; and with the thickness of the selected film.

## Claims

1. A method for supplying a microbial activity controlling material which comprises: filling a microbial activity controlling material into a vessel having a sealed structure and at least a part of which is provided with a non-porous film, supplying the microbial activity controlling material through the non-porous film part of the vessel to ambient region around the vessel at the speed controlled by the molecular permeation performance of the non-porous film, and thereby controlling the activities of microorganisms residing around the vessel.

2. The method for supplying a microbial activity controlling material according to Claim 1, wherein the microbial activity controlling material is a material which functions as electron donor which is an energy source of microorganism, and the method controls the activity of a microorganism which needs the electron donor among the microorganisms residing in the ambient region around the vessel.

3. The method for supplying a microbial activity controlling material according to Claim 1, wherein the microbial activity controlling material is an acidic material or a basic material, and the method controls the activities of the microorganisms by controlling pH at the ambient region around the vessel.

4. The method for supplying a microbial activity controlling material according to Claim 1, wherein the microbial activity controlling material is an inorganic salt, and the method controls the activities of the microorganisms by enhancing the concentration of the inorganic salt at the ambient region around the vessel.

5. The method for supplying a microbial activity controlling material according to Claim 1, wherein the microbial activity controlling material is an oxygen releasing material, and the method controls the activity of an aerobic microorganism among the microorganisms by supplying oxygen to the ambient region around the vessel.

6. The method for supplying a microbial activity controlling material according to Claim 1, wherein the microbial activity controlling material is an oxygen absorbing material, and the method controls the activity of an anaerobic microorganism among the microorganisms by absorbing oxygen to the ambient region around the vessel.

7. A microbial activity controlling material supplying apparatus which comprises: a microbial activity controlling material and a vessel having a sealed structure and at least a part of which is provided with a non-porous film, wherein the vessel is filled with the microbial activity controlling material, and releases the microbial activity controlling material through the non-porous film part of the vessel to ambient region around the vessel at the speed controlled by the molecular permeation performance of the non-porous film.

8. The microbial activity controlling material supplying apparatus according to Claim 7, wherein the microbial activity controlling material is at least one selected from a group of materials which function as electron donor , acidic materials, basic materials, inorganic salts, oxygen releasing materials and oxygen absorbing materials, wherein combinations of the acidic materials and the basic materials and combinations of the oxygen releasing materials and the oxygen absorbing materials are omitted.

9. The microbial activity controlling material supplying apparatus according to Claim 8, wherein the material which functions as the electron donor is one or more members selected from a group of hydrogen, hydrogen sulfide and organic compounds permeable through the non-porous film.

10. The microbial activity controlling material supplying apparatus according to Claim 8, wherein waste alcohol is used as the material which functions as the electron donor.

11. The microbial activity controlling material supplying apparatus according to Claim 7, wherein the vessel is a bag or tube in which the microbial activity controlling material is sealed.

12. The microbial activity controlling material supplying apparatus according to Claim 7, wherein the vessel further comprises a supply part for refilling the microbial activity controlling material.

13. The microbial activity controlling material supplying apparatus according to Claim 12, wherein the supply part comprises a tank part, to which the microbial activity controlling material is temporary reserved, and the supply part is integrated with the vessel.

14. The microbial activity controlling material supplying apparatus according to Claim 12, wherein the vessel further comprises a supply nozzle which is connected with a reservoir tank for reserving the microbial activity controlling material which is formed separately from the vessel, and which is possible to refill the microbial activity controlling material as occasion demands.

15. The microbial activity controlling material supplying apparatus according to Claim 7, wherein a protective member for protecting the non-porous film from external impacts is provided on a surface of the non-porous film.

16. The microbial activity controlling material supplying apparatus according to Claim 7, wherein a carrier which is able to immobilize microorganisms is provided on a surface of the non-porous film.

17. The microbial activity controlling material supplying apparatus according to Claim 7, wherein the non-porous film is a hydrophobic film.

18. The microbial activity controlling material supplying apparatus according to Claim 17, wherein the hydrophobic film is one of polyethylene v and polypropylene film.

19. The microbial activity controlling material supplying apparatus according to Claim 7, wherein the non-porous film is a hydrophilic film.

20. The microbial activity controlling material supplying apparatus according to Claim 19, wherein the hydrophilic film is polyvinyl alcohol film.

21. The microbial activity controlling material supplying apparatus according to Claim 7, wherein the non-porous film is an amphipathic film.

22. The microbial activity controlling material supplying apparatus according to Claim 21, wherein the amphipathic film is ethylene - vinyl alcohol copolymer v.

23. An environmental purification method for removing environmental pollutant which comprises: placing the microbial activity controlling material supplying apparatus according to Claim 7 in a decontamination targeting area, supplying the microbial activity controlling material to the ambient region around the microbial activity controlling material supplying apparatus, and controlling the activity of the microorganisms residing at the ambient region around the microbial activity controlling material supplying apparatus.

24. A soil improving method for improving exhausted soil which comprises: burying the microbial activity controlling material supplying apparatus according to Claim 7 in exhausted soil, supplying the microbial activity controlling material to the ambient region around the microbial activity controlling material supplying apparatus, and controlling the activity of the microorganisms residing at the ambient region around the microbial activity controlling material supplying apparatus.

25. The environmental purification method according to Claim 23, wherein a material which functions as electron donor is used as the microbial activity controlling material, and the electron donor is supplied to the microorganisms residing at the ambient region around the microbial activity controlling material supplying apparatus, and the activity of a microorganism which needs the electron donor among the microorganisms residing in the ambient region around the microbial activity controlling material supplying apparatus is activated to remove the environmental pollutant.

26. The environmental purification method according to Claim 23, wherein an oxygen releasing material is used as the microbial activity controlling material, and the oxygen is supplied to the microorganisms residing at the ambient region around the microbial activity controlling material supplying apparatus, and the activity of an aerobic microorganism among the microorganisms residing in the ambient region around the microbial activity controlling material supplying apparatus is activated to remove the environmental pollutant.

27. The environmental purification method according to Claim 25 or Claim 26, wherein a carrier which is able to immobilize microorganisms is provided on a surface of the non-porous film, and a microorganism which degrade the environmental pollutant is immobilized on the carrier in advance.

28. An environmental purification method for removing environmental pollutant which comprises: placing, as a first microbial activity controlling material supplying apparatus, the microbial activity controlling material supplying apparatus according to Claim 7 and which is filled with a material which functions as electron donor in a decontamination targeting area, while placing, as a second microbial activity controlling material supplying apparatus, another microbial activity controlling material supplying apparatus according to Claim 7 and which is filled with an oxygen absorbing material at a location where an anaerobic circumstance is formed by absorbing oxygen from the ambient region around the first microbial activity controlling material supplying apparatus, and activating an anaerobic microorganism which needs the electron donor among the microorganisms residing in the ambient region around the first microbial activity controlling material supplying apparatus.

29. An environmental purification method for removing environmental pollutant which comprises: placing, as a first microbial activity controlling material supplying apparatus, the microbial activity controlling material supplying apparatus according to Claim 7 and which is filled with a material which functions as electron donor in a decontamination targeting area, while placing, as a second microbial activity controlling material supplying apparatus, another microbial activity controlling material supplying apparatus according to Claim 7 and which is filled with an oxygen releasing material at a location where an aerobic circumstance is formed and near the first microbial activity controlling material supplying apparatus, and activating an aerobic microorganism which needs the electron donor among the microorganisms residing in the ambient region around the first microbial activity controlling material supplying apparatus.

30. An environmental purification method for removing environmental pollutant which comprises: placing, as a first microbial activity controlling material supplying apparatus, the microbial activity controlling material supplying apparatus according to Claim 7 and which is filled with a material which functions as electron donor in a decontamination targeting area; while placing, as a second microbial activity controlling material supplying apparatus, another microbial activity controlling material supplying apparatus according to Claim 7 and which is filled with an oxygen releasing material at a location where oxygen is not supplied and which is near the first apparatus, so that a material produced by an activated aerobic microorganisms residing at the ambient region around the second activity controlling material supplying apparatus is supplied to other microorganisms residing at the ambient region around the first activity controlling material supplying apparatus; and thus removing the environmental pollutant by a microorganism which needs the electron donor among the microorganisms residing in the ambient region around the first microbial activity controlling material supplying apparatus and an aerobic microorganism among the microorganisms residing in the ambient region around the second microbial activity controlling material supplying apparatus.

31. An environmental purification method for removing environmental pollutant which comprises: placing, as a first microbial activity controlling material supplying apparatus, the microbial activity controlling material supplying apparatus according to Claim 7 and which is filled with a material which functions as electron donor in a decontamination targeting area; placing, as a second microbial activity controlling material supplying apparatus, another microbial activity controlling material supplying apparatus according to Claim 7 and which is filled with an oxygen releasing material at a location where oxygen is not supplied and which is near the first apparatus, so that a material produced by an activated aerobic microorganisms residing at the ambient region around the second activity controlling material supplying apparatus is supplied to other microorganisms residing at the ambient region around the first activity controlling material supplying apparatus; and further placing, as a third microbial activity controlling material supplying apparatus, still other microbial activity controlling material supplying apparatus according to Claim 7 and which is filled with an oxygen absorbing material at a location where oxygen is not supplied from the second microbial activity controlling material supplying apparatus and where an anaerobic circumstance is formed by absorbing oxygen from the ambient region around the first microbial activity controlling material supplying apparatus; and thus removing the environmental pollutant by an anaerobic microorganism which needs the electron donor among the microorganisms residing in the ambient region around the first microbial activity controlling material supplying apparatus and an aerobic microorganism among the microorganisms residing in the ambient region around the second microbial activity controlling material supplying apparatus.

32. A bioreactor which comprises a carrier onto which microorganisms which is effective in the removal of a target component are immobilized is arranged around the non-porous film part of the microbial activity controlling material supplying apparatus according to Claim 7.

33. The bioreactor according to Claim 32, wherein the carrier is attached and stuck on the non-porous film parts.

34. The bioreactor according to Claim 32, wherein the carrier is formed as a bag, and the microbial activity controlling material supplying apparatus according to Claim 7 is installed in an interior space of the bag.

35. The bioreactor according to Claim 32, wherein the carrier comprises a water absorbent polymer.

36. A bioreactor which comprises a microorganism which is directly carried on a surface of the non-porous film of the microbial activity controlling material supplying apparatus according to Claim 7.

37. The bioreactor according to Claim 32, wherein one or more kinds of microorganisms which are effective for removing a target material from a region to be treated, the region being in one of liquid phase, gaseous phase and solid phase, and one or more kinds of microorganisms which oxidize or reduce the material produced by the former microorganisms, are immobilized to the carrier, and the region to be treated is subjected to contact with one face of the carrier while a material which functions as electron donor is filled to contact with the other face of the carrier.

38. The bioreactor according to Claim 37, wherein the microorganism which is effective for removing a target material from the region to be treated is an ammonia-oxidizing bacterium, and the microorganism which reduces the material produced by the former microorganism is a denitrifying bacterium.
